Europäisches Patentamt

European Patent Office

Office européen des brevets

(11) Publication number : **0 497 527 A1**

## (12) EUROPEAN PATENT APPLICATION

(21) Application number : **92300660.5**

(22) Date of filing : **27.01.92**

(51) Int. Cl.⁵ : **C12Q 1/68,** C12P 19/34,
// C07H21/04

(30) Priority : **31.01.91 GB 9102148**
**06.12.91 GB 9126085**

(43) Date of publication of application :
**05.08.92 Bulletin 92/32**

(84) Designated Contracting States :
**DE FR GB IT NL SE**

(71) Applicant : **IMPERIAL CHEMICAL**
**INDUSTRIES PLC**
**Imperial Chemical House, Millbank**
**London SW1P 3JF (GB)**

(72) Inventor : **Little, Stephen**
**ICI Cellmark Diagnostics, Gadbrook Park**
**Rudheath, Northwich, Cheshire CW9 7RA (GB)**
Inventor : **Ferrie, Richard Mark**
**ICI Cellmark Diagnostics, Gadbrook Park**
**Rudheath, Northwich, Cheshire CW9 7RA (GB)**
Inventor : **Robertson, Nancy Hastings**
**ICI Cellmark Diagnostics, Gadbrook Park**
**Rudheath, Northwich, Cheshire CW9 7RA (GB)**

(74) Representative : **Phillips, Neil Godfrey Alasdair**
**et al**
**ICI Group Patents, Group Patents Services**
**Department, PO Box 6, Shire Park, Bessemer**
**Road**
**Welwyn Garden City, Hertfordshire AL7 1HD**
**(GB)**

(54) Detection method for nucleotide sequences.

(57) A method for detecting the presence or absence of more than one variant nucleotide in a target base sequence comprised in a nucleic acid sample using diagnostic primers having a terminal nucleotide complementary to either a suspected variant nucleotide or the corresponding normal nucleotide in the target base sequence and wherein the extension products of more than one diagnostic primer comprise a complementary overlap. Primers and kits for use in the claimed method.

Fig.10(i)

Fig.10(ii)

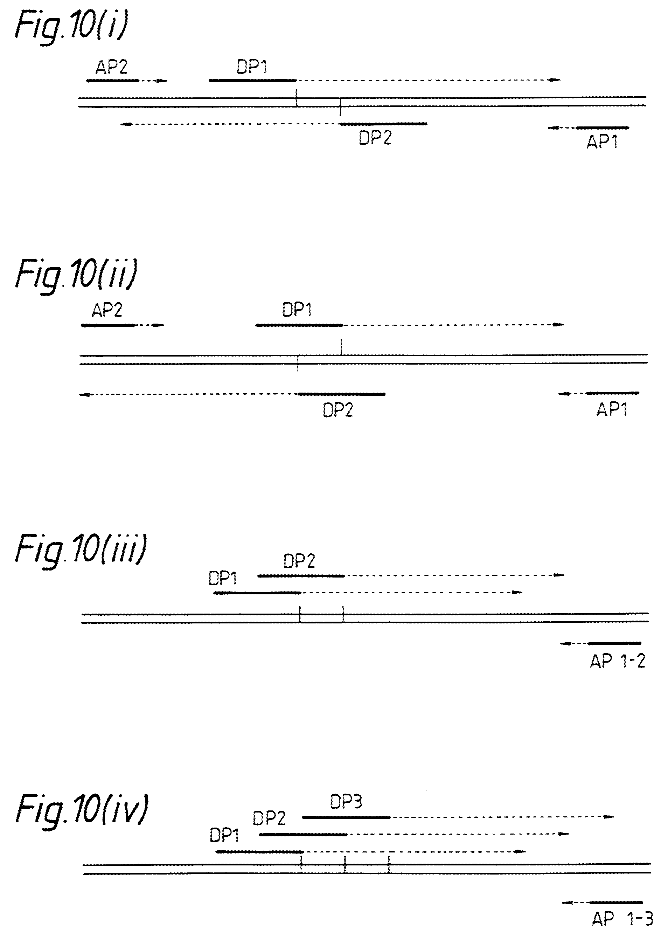

Fig.10(iii)

Fig.10(iv)

EP 0 497 527 A1

The present invention relates to a method for detecting the presence or absence of at least one variant nucleotide in a target base sequence in a nucleic acid sample by amplification or the absence thereof and kits therefor.

The present invention is of particular interest in the diagnostic screening of DNA samples for inherited conditions, predispositions or somatic mutations and provides inter alia a general method for the facile detection of point mutations. It is also useful in the detection and typing of infectious pathogens by analysis of their DNA or RNA.

Several hundred genetic diseases are known to exist in man which result from particular mutations at the DNA level. The molecular basis for certain of these diseases is already known and research is rapidly revealing the molecular basis for those genetic diseases for which the nature of the mutation is at present unknown. Where the precise molecular basis for the inherited condition is not known, diagnosis of the disorder or location of carriers may be provided in informative pedigrees by RFLP technology using DNA probes in genetic linkage with the disease locus. Thus, at present Duchenne Muscular Dystrophy, Cystic Fibrosis and Huntington's Chorea inter alia may for example be diagnosed using RFLP technology. Such testing however needs to be performed separately in respect of each condition and a substantial amount of work is required, each case requiring inter alia DNA purification, restriction enzyme digestion, agarose gel electrophoresis, Southern blotting, hybridisation, detection of hybridised gene probe and pedigree analysis. Certain other inherited conditions are known to be associated with single point mutations in genes, but each of these conditions must be analysed separately and further particular difficulties arise where the point mutations are heterogeneous. Thus for example more than 40 different point mutations can cause β- thalassaemia and at least 5, and probably many more than 12, point mutations can cause haemophilia A. In respect of these heterogeneous conditions, each potential mutation point may need at present to be analysed separately. This can involve complex RFLP haplotype analysis with multiple restriction enzymes.

A number of point mutations in somatic cells have been implicated in the development of various cancers for example point mutations within the ras oncogene (J.L. Boos et al, Nature 327, 293 (1987).

In our European Patent Application, Publication No. 0332435, the contents of which are incorporated herein by reference, we describe and claim the Amplification Refractory Mutation System (ARMS). ARMS is a method for detecting the presence or absence of at least one variant nucleotide in one or more nucleic acids contained in a sample, which method comprises treating the sample, together or sequentially with appropriate nucleoside triphosphates, an agent for polymerisation of the nucleoside triphosphates and a diagnostic primer for a diagnostic portion of a target base sequence under hybridising conditions, the nucleotide sequence of the said diagnostic primer being such that it is substantially complementary to the said diagnostic portion, a terminal nucleotide of the diagnostic primer being either complementary to the suspected variant nucleotide or to the corresponding normal nucleotide, whereby an extension product of the diagnostic primer is synthesised when the said terminal nucleotide of the diagnostic primer is complementary to the corresponding nucleotide in the target base sequence, no extension product being synthesised when the said terminal nucleotide of the diagnostic primer is not complementary to the corresponding nucleotide in the target base sequence; and detecting the presence or absence of the suspected variant nucleotide from the presence or absence of an extension product.

ARMS is preferably effected with a corresponding amplification primer, any extension product of the diagnostic primer formed being capable of serving as a template for synthesis of an extension product of the amplification primer after separation from its complement. Amplification using both diagnostic and amplification primers may then be effected to provide, where an extension product has been synthesised, an appropriate number of copies for detection.

A further and important use of ARMS, as described in our European Patent Application, Publication No. 0332435, is for detecting the presence or absence of more than one suspected variant nucleotide in the same sample. The ability of ARMS to selectively amplify sequences depending on the predetermined nucleotide sequence of the diagnostic primers enables multiple amplification products to be distinguished simply, accurately and with minimal operator skill thus making it possible to provide a robust technique for screening a single sample for multiple nucleotide variations. The use of ARMS to detect more than one suspected variant nucleotide in the same sample is conveniently referred to as multiplex ARMS. Multiplex ARMS is thus of particular interest in screening a single sample of DNA or RNA for a battery of inherited conditions such as genetic disorders, predispositions and somatic mutations leading to various diseases. Such DNA or RNA may for example be extracted from blood or tissue material such as chorionic villi or amniotic cells by a variety of techniques such as those described by Maniatis et al, Molecular Cloning (1982), 280-281. Morever as the molecular basis for further inherited conditions becomes known these further conditions may simply be included in the screening technique of the present invention.

Multiple amplification products may be distinguished by a variety of techniques. Thus for example probes may be employed for each suspected amplified product, each probe carrying a different and distinguishable signal or residue capable of producing a signal.

A much simpler and preferred method of distinguishing between ARMS amplification products comprises selecting the nucleotide sequences of the amplification primers such that the length of each amplified product formed during the process of the present invention is different. In this regard the number of base pairs present in an amplification product is dictated by the distance apart of the diagnostic and amplification primers. Thus the amplification primers may be designed such that each potential variant nucleotide is associated with a potential amplification product of different length.

The present invention is based on the discovery that mulitplex ARMS may be successfuly performed where diagnostic primer extension products of more than one diagnostic portion of the target base sequence comprise a complementary overlap. This unexpected improvement to multiplex ARMS now facilitates the detection and analysis of, for example, inherited or infectious disease where the potential variant nucleotides are closely spaced.

Therefore according to a first aspect of the present invention we provide a method for detecting the presence or absence of more than one variant nucleotide in a target base sequence comprised in a nucleic acid sample, which method comprises treating the target base sequence, together or sequentially with appropriate nucleoside triphosphates, an agent for polymerisation of the nucleoside triphosphates and a diagnostic primer for each diagnostic portion of the target base sequence under hybridising conditions, the nucleotide sequence of each diagnostic primer being such that it is substantially complementary to the relevant diagnostic portion, a terminal nucleotide of each diagnostic primer being either complementary to a suspected variant nucleotide or to the corresponding normal nucleotide, whereby an extension product of a diagnostic primer is synthesised when the terminal nucleotide of the diagnostic primer is complementary to the corresponding nucleotide in the target base sequence, no extension product being synthesised when the terminal nucleotide of a diagnostic primer is not complementary to the corresponding nucleotide in the target base sequence; and detecting the presence or absence of the suspected variant nucleotides from the presence or absence of extension products and wherein the extension products of diagnostic primer(s) for more than one diagnostic portion of the target base sequence comprise a complementary overlap.

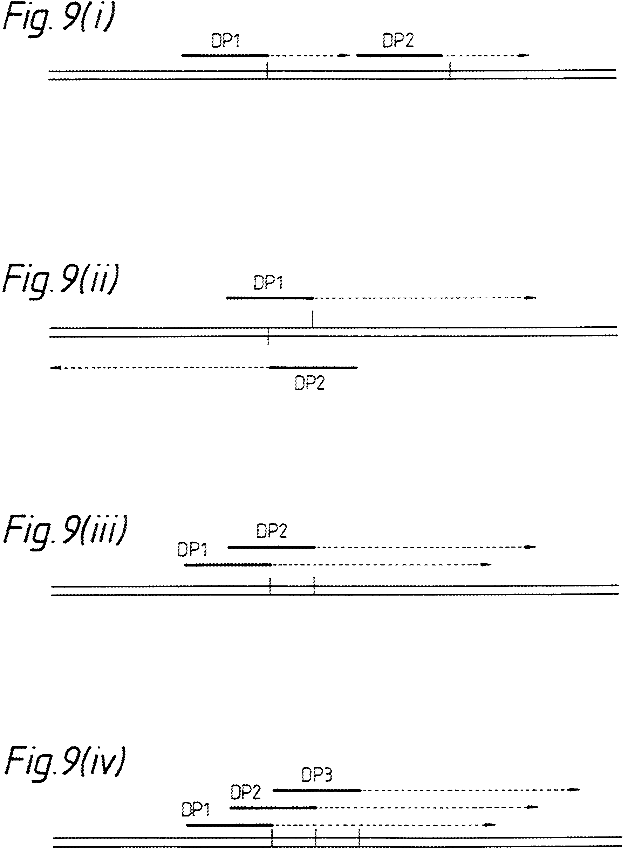

The overlap may occur due to any convenient arrangement of the diagnostic primers on the target base sequence, for example as illustrated in Figure 9 (i).

Furthermore, we have unexpectedly found that ARMS may be successfully performed where the diagnostic primer(s) for more than one diagnostic portion of the target base sequence comprise a complementary overlap, for example as illustrated in Figure 9 (iii) or (iv).

The design of appropriate diagnostic primers will, in general be determined by the nature and position of the relevant variant nucleotides. In addition, the molecular biologist of ordinary skill may determine convenient and preferred primers and reaction conditions, for example by routine experimentation and in particular by reference to the examples provided in this patent application.

It will further be appreciated that any diagnostic primer extension product(s) obtained may if desired be amplified by known methods such as the polymerase chain reaction (PCR) as described in U.S.Patent Nos.4683195 and 4683202, by the use of Q-beta replicase as described in PCT Patent Publication WO87/06270 and in Biotechnology Vol.6 October 1988, by the use of the transcription based nucleic acid amplification of Siska Corporation as described in PCT Patent Publication WO88/10315, or by the use of linear or arithmetical amplification. The expression "linear or arithmetical amplification" refers to repeated cycles of primer annealing, extension and separation using the same nucleic acid template. Linear amplification is more fully described in our European Patent Application, Publication No. 0332435.

If required the target nucleic acid may be amplified, for example using any of the methods mentioned above, prior to performing the method of the invention.

The method of the invention is preferably effected with a corresponding amplification primer, any extension product of a diagnostic primer being capable of serving as a template for synthesis of an extension product of the amplification primer after separation from its complement. This is conveniently illustrated in Figure 10 (iii) and (iv). A single amplification primer is used for all diagnostic primers.

In our European patent application, publication no. 0332435 we disclose that ARMS may be effected using either

(a) a first diagnostic primer having a sequence substantially complementary to a diagnostic portion of a first nucleic acid sequence, the first diagnostic primer having a terminal nucleotide complementary to the said suspected variant nucleotide, and a second diagnostic primer having a sequence substantially complementary to a diagnostic portion of a second nucleic acid sequence, the second diagnostic primer having a terminal nucleotide complementary to the complementary suspected variant nucleotide; or

(b) a first diagnostic primer having a sequence substantially complementary to a diagnostic portion of a

3

first nucleic acid sequence, the first diagnostic primer having a terminal nucleotide complementary to the normal nucleotide which corresponds to the said suspected variant nucleotide, and a second diagnostic primer having a sequence substantially complementary to a diagnostic portion of a second nucleic acid sequence, the second diagnostic primer having a terminal nucleotide complementary to the normal nucleotide which corresponds to the said suspected variant nucleotide; the said terminal nucleotide of the first diagnostic primer and the said terminal nucleotide of the second diagnostic primer being either both at the 5′ end or both at the 3′ end of the respective primers and the first nucleic acid sequence being in the opposite sense to the second nucleic acid sequence. In the above embodiment of ARMS, the second diagnostic primer may act as an amplification primer.

We have now unexpectedly found that, where ARMS is effected with diagnostic primers for diagnostic portions of first and second target base sequences, corresponding amplification primers may be provided whereby the amplification products of at least two diagnostic primers and their corresponding amplification primers comprise a complementary overlap. This is conveniently illustrated in Figure 10 (i).

We have also unexpectedly found that any of the diagnostic and/or amplification primers conveniently the diagnostic primers for diagnostic portions of first and second target base sequences may comprise a complementary overlap, for example as illustrated in Figure 9 (ii) and Figure 10 (ii).

The method of the invention is conveniently used for HLA typing, in the diagnosis of β- thalasaemia, sickle cell anaemia, phenylketonuria (PKU), Factor VIII and IX blood disorders and α-1-antitrypsin

A particular use for the method of the present invention is in the detection and diagnosis of cystic fibrosis. Convenient cystic fibrosis alleles are disclosed in our European Patent Application No. 90309420.9; by B. Kerem et al, Science, 1989, 245, 1073-1080; by J.R. Riordan et al, Science, 1989, 245, 1066-1073; by J.M. Rommens et al, Science, 1989, 245, 1059-1065; by G.R. Cutting et al, Nature, 346, 366-368; by M. Dean et al, Cell, 61, 863-870; by K. Kobayashi et al, Am. J. Hum. Genet., 1990, 47, 611-615; by B. Kerem et al, Proc. Natl. Acad. Sci. USA, 1990, 87, 8447; by M. Vidaud et al, Human Genetics, 1990, 85, (4), 446-449; and by M.B. White et al, Nature, 344, 665-667. Table 4 set out hereinafter shows convenient CF mutations.

If only 1, 2 or 3 nucleoside triphosphates are present then a diagnostic primer will only extend as far as the presence of these nucleoside triphosphates will permit. As indicated above, where there is a mismatch between for example the 3′ terminal end of the diagnostic primer and the corresponding nucleoside triphosphate in the sample nucleic acid no primer extension will be effected . Where, however, the 3′ terminal nucleoside triphosphate is complementary with the corresponding nucleoside triphosphate in the sample nucleic acid, primer extensions will be effected.

Where only 1, 2 or 3 nucleoside triphosphates are used and in use, the terminal nucleoside triphosphate of the extended diagnostic primer is only employed once, then it may be advantageous to use a dideoxy nucleoside triphosphate as the nucleoside triphosphate which in use will constitute the terminal nucleoside triphosphate of the diagnostic primer extended product. This will assist in production of a clearly terminated extension product of a diagnostic primer.

If desired one or more of the nucleoside triphosphates present in the reaction mixture for the purpose of incorporation into the extended primer(s) may be labelled or marked in any convenient manner. Thus for example one or more of the nucleoside triphosphates may be fluorescently labelled. This labelling of the nucleoside triphsophates is of particular interest where production of an extension product of a diagnostic primer can be detected by detection of the labelled or marked nucleoside triphosphate(s) incorporated in the extension product. Where no extension product is formed no incorporation takes place, and the labelled or marked nucleoside triphosphates may for example be washed away.

More particularly this avoids the problem of amplification of artefactual products and thus enables good discrimination to be achieved in the presence of the labelled or marked nucleoside triphosphate(s). Where amplification is effected for example by the use of PCR any production of an artefactual product may result in amplification of that product and thus incorporation of the labelled or marked nucleoside triphosphate thereby reducing discrimination.

In addition to the above it may be desirable that the diagnostic primer carry one member of an immunological binding pair, for example an antigen or an antibody, or one member of a complex forming pair, for example biotin, for binding to the other member of said binding pair or forming pair for the purpose of capture on to solid phase.

Whilst we do not wish to be bound by any theoretical considerations it is believed that the method of the present invention may be performed using any convenient number of diagnostic primers, such as up to 50, up to 40, up to 30 , up to 25, for example up to 20, up to 15, up to 10, 9, 8, 7, 6, 5, 4, or up to 3 diagnostic primers.

According to a further feature of the present invention there is provided a kit for detecting the presence or absence of more than one variant nucleotide in a target base sequence comprised in a nucleic acid sample, which kit comprises:-

(1) a diagnostic primer for each diagnostic portion of the target base sequence, the nucleotide sequence of each diagnostic primer being such that it is substantially complementary to the relevant diagnostic portion, a terminal nucleotide of a diagnostic primer being either complementary to a suspected variant nucleotide or to the corresponding normal nucleotide such that in use an extension product of a diagnostic primer is synthesised when the terminal nucleotide of the diagnostic primer is complementary to the corresponding nucleotide in the target base sequence, no extension product being synthesised when the terminal nucleotide of the diagnostic primer is not complementary to the corresponding nucleotide in the target base sequence and wherein the potential extension products of diagnostic primers for more than one diagnostic portion of the target base sequence comprise a complementary overlap;

(2) each of four different nucleoside triphosphates; and

(3) an agent for polymerisation of the nucleoside triphosphates in (2).

The normal and variant primers for each diagnostic portion of the target base sequence may be provided in any convenient number of containers, such as tubes. By way of example all the normal primers and all the variant primers are provided in two separate tubes. Alternatively three tubes are provided with any convenient combination of normal and variant primers comprised therein.

Advantageously the kit of the present invention additionally comprises corresponding amplification primer(s), the nucleotide sequence of the amplification primer being such that any extension product of a corresponding diagnostic primer may, after separation from its complement, serve as a template for synthesis of an extension product of the amplification primer. The kit of the present invention may also, if desired, include internal control primers, where appropriate.

It is especially preferred, however, that the kit of the present invention comprises PCR (polymerase chain reaction) primer(s) and a diagnostic primer (as hereinafter defined) in respect of each suspected variant nucleotide.

Each of the materials detailed in (1), (2) and (3) and/or the amplification primer may be conveniently packaged in a separate container, but preferably all may be combined in a single container to which the material to be analysed is added. Advantageously the single container will additionally contain buffer.

Printed instructions for the use of a kit as claimed above are also optionally provided.

A particular use for the kit of the present invention is in the detection and diagnosis of cystic fibrosis.

The term "nucleoside triphosphate" is used herein to refer to nucleosides present in either DNA or RNA and thus includes nucleosides which incorporate adenine, cytosine, guanine, thymine and uracil as base, the sugar moiety being deoxyribose or ribose. In general deoxyribonucleosides will be employed in combination with a DNA polymerase. It will be appreciated however that other modified bases capable of base pairing with one of the conventional bases adenine, cytosine, guanine, thymine and uracil may be employed. Such modified bases include for example 8-azaguanine and hypoxanthine.

The term "nucleotide" as used herein can refer to nucleotides present in either DNA or RNA and thus includes nucleotides which incorporate adenine, cytosine, guanine, thymine and uracil as base, the sugar moiety being deoxyribose or ribose. It will be appreciated however that other modified bases capable of base pairing with one of the conventional bases, adenine, cytosine, guanine, thymine and uracil, may be used in the diagnostic primer and amplification primer employed in the present invention. Such modified bases include for example 8-azaguanine and hypoxanthine.

It will be appreciated that where the method of the present invention is to be used for detecting the presence or absence of suspected variant nucleotides adjacent to portions of the target base sequence which do not contain all four different nucleotides, then extension products of the diagnostic primers and, if desired, extension products of the amplification primers may be formed in the presence of only the appropriate corresponding nucleoside triphosphates and all four different nucleoside triphosphates would not be necessary.

The agent for polymerization of the nucleoside triphosphates may be any compound or system which will function to accomplish the synthesis of primer extension products, including enzymes. Suitable enzymes for this purpose include, for example, E.coli DNA Polymerase I, Klenow fragment of E.coli DNA polymerase I, T4 DNA polymerase, other available DNA polymerases, reverse transcriptase, and other enzymes, including thermostable enzymes. The term "thermostable enzyme" as used herein refers to an enzyme which is stable to heat and is heat resistant and catalyzes (facilitates) combination of the nucleotides in the proper manner to form the primer extension products which are complementary to each nucleic acid strand. Generally, the synthesis will be initiated at the 3′ end of each primer and will proceed in the 5′ direction along the template strand, until synthesis terminates, producing molecules of different lengths. There may be enzymes for example, thermostable enzymes, however, which initiate synthesis at the 5′ end and proceed in the other direction, using the same process as described above. A preferred thermostable enzyme which may be employed in the process of the present invention is that which can be extracted and purified from Thermus aquaticus. Such enzyme has a molecular weight of about 86,000 - 90,000 daltons as described in European Patent Publication No.

237,362 (see also European Patent Publication No 258,017). Thermus aquaticus strain YT1 is available without restriction from the American Type Culture Collection, 12301 Parklawn Drive, Rockville, Maryland, USA as ATCC 25,104.

The expression "diagnostic portion" as used herein means that portion of the target base sequence (as hereinafter defined) which contains as its terminal nucleotide the suspected variant nucleotide(s), the presence or absence of which is/are to be detected. Generally the suspected variant nucleotide will be at the 5'-terminal end of the diagnostic portion since in general synthesis of primer extension products will be initiated at the 3' end of each primer as described above. Where however an agent for polymerisation is to be used which initiates synthesis at the 5' end of the diagnostic primer and proceeds in the 3' direction along the template strand until synthesis terminates the "diagnostic portion" will contain the suspected variant nucleotide at its 3' end. The diagnostic primers will also be appropriately designed in this regard as set out below.

The expression "target base sequence" as used herein means a nucleotide sequence comprising more than one diagnostic portion (as hereinbefore defined). It will be appreciated that a nucleic acid duplex, such as a DNA duplex comprises both a first and a second target base sequence. The target base sequence will generally depend on, for example, the inherited or acquired disease to be analysed. Thus for example in respect of cystic fibrosis the target base sequence is about 80 nucleotides in length. Examples of convenient target base sequences include those of up to one kilobase, such as up to 900 bases, up to 800 bases, up to 700 bases, up to 600 bases, up to 500 bases, up to 400 bases, up to 350 bases, for example up to 300 bases, up to 250 bases, up to 200 bases, up to 150 bases or more particularly up to 100 bases, such as up to 80, 60 or 40 bases.

The term "primer" as used herein refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of appropriate nucleoside triphosphates and an agent for polymerisation such as DNA polymerase in an appropriate buffer ("buffer" includes pH, ionic strength, cofactors, etc.) and at a suitable temperature. The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the agent for polymerization. The exact lengths of the primers will depend on many factors, including temperature and source of primer and use of the method. For example, depending on the complexity of the target sequence, the diagnostic and amplification primers typically contain 12-35, for example, 15-35 nucleotides, although they may contain more or fewer nucleotides. Short primer molecules generally require lower temperatures to form sufficiently stable hybrid complexes with the template.

The term "oligonucleotide" as used herein is defined as a molecule comprised of two or more deoxyribonucleotides or ribonucleotides, preferably more than three. Its exact size will depend on many factors, such as the reaction temperature, salt concentration, the presence of formamide and the presence of other close mutation(s), such as in sickle cell Hb C disease, which in turn depend on the ultimate function or use of the oligonucleotide. Indeed, the exact sequence of the oligonucleotide may also depend on a number of factors as described hereinafter. The oligonucleotide may be derived synthetically or by cloning.

The term "extension products" as used herein is defined as the polynucleotides which may or may not be formed, depending on the presence or absence of variant nucleotide(s) in a diagnostic portion of the target base sequence, by diagnostic primer extension on the target base sequence template.

The term "amplification products" as used herein is defined as the polynucleotides which may or may not be formed, depending on the presence or absence of variant nucleotide(s) in a diagnostic portion of the target base sequence, by diagnostic primer extension followed by corresponding amplification primer extension on the diagnostic primer extension product.

The term "complementary overlap" as used herein is defined, in respect of both primers and extension products, as a region of the target base sequence to which at least two of the relevant primers or two of the relevant extension products, from different diagnostic regions of the target base sequence, selectively hybridise in the method of the invention. This is conveniently illustrated with reference to Figure 9 (i), (iii) and (iv) where extension products from different diagnostic regions of the target base sequence are indicated by broken lines. In respect of amplification products from diagnostic/amplification primers on first and second target base sequences, for example as indicated in Figure 10 (i) and (ii), the complementary overlap is defined as a region of the target base sequence common to the amplification products. The degree of overlap depends on the primers selected. Any convenient degree of overlap may be employed such as up to 90%, up to 80%, up to 70%, up to 60%, up to 50%, up to 40%, up to 30%, 20%, or up to 10% overlap. It will be understood that in respect of primer extension products the complementary overlap only arises between primer extension products used in diagnosis and not as the result of mispriming. Such products are generally of up to 5kb, in particular up to 2kb, up to 1.5kb, up to 1kb, up to 900 bases, up to 800 bases, up to 700 bases, up to 600 bases, conveniently up to 500 bases, up to 400 bases, up to 300 bases, up to 200 bases or up to 100 bases.

The term "complementary to" is used herein in relation to nucleotides to mean a nucleotide which will base pair with another specific nucleotide. Thus adenine is complementary to uricil or thymine and guanine is complementary to cytosine. It is appreciated that whilst thymidine and guanidine may base pair under certain circumstances they are not regarded as complementary for the purposes of this specification. It will also be appreciated that whilst cytosine and adenine may base pair under certain circumstances they are not regarded as complementary for the purposes of this specification. The same applies to cytosine and uracil.

The primers herein are selected to be "substantially" complementary to the different strands of each specific sequence to be amplified. This means that the primers must be sufficiently complementary to hybridize with their respective strands. Therefore, the primer sequence need not reflect the exact sequence of the template. For example, where the diagnostic primer comprises a nucleotide sequence in which the 3'-terminal nucleotide is complementary to either the suspected variant nucleotide or the corresponding normal nucleotide a non-complementary nucleotide fragment may be attached to the 5'-end of the primer, with the remainder of the primer sequence being complementary to the diagnostic portion of the target base sequence. Commonly, however, the primers have exact complementarity except in so far as non-complementary nucleotides may be present at a pre-determined primer terminus as hereinbefore described.

It will be appreciated, however, that in certain circumstances synthesis of a diagnostic primer extension product might be induced to occur even in the presence of a non-complementary 3'-terminal residue. This artefactual result may arise from the use of too low a temperature in which case the temperature may be increased, too long a time of incubation/annealing in which case the time may be reduced, too high a salt concentration in which case the salt concentration may be reduced, too high an enzyme concentration, too high a nucleoside triphosphate concentration, an incorrect pH or an incorrect length of oligonucleotide primer. All of these factors are discussed in European Patent Publication No 237,362. A major source of artefactual products is probably allowing the reaction temperature to fall too low, thus permitting too low a stringency, for example by removing the reaction mixture from the heat cycling means, even briefly for example to add the agent for polymerisation (eg. Taq polymerase) especially in the first reaction cycle. In addition to the above we have found that such artefactual results may also arise from use of a diagnostic primer which is particularly rich in G (guanosine) and C(cytidine) residues. A diagnostic primer may give rise to difficulty in this regard if it is G/C rich as a whole or particularly if it is G/C rich at its relevant, normally, 3', end. Moreover the precise nature of the base pairing in the region of the relevant, normally 3', end of the diagnostic primer when in use may be the cause of an artefactual result. Thus the presence of As (adenosine) in the base pairing in the region of the relevant, normally 3', end of the diagnostic primer tends to improve specificity whilst the presence of Gs (guanosine) does not. Furthermore the precise nature of the mismatch at the relevant, normally 3', end of the diagnostic primer may be an important factor in whether or not an artefactual result is obtained. Thus for example an AA or CT mismatch does not normally result in hybridisation, but a GT or AC mismatch may result in a sufficient degree of hybridisation to result in the formation of artefactual product(s). Artefactual results may be avoided by deliberately introducing one or more further mismatched residues, or if desired, deletions or insertions, within the diagnostic primer to destabilise the primer by further reducing the binding during hybridisation.

Thus for example any one or more of the 10, for example 6 nucleotides adjacent to the terminal mismatch may be altered to introduce further mismatching. In general only one mismatch in addition to the terminal mismatch may be necessary, positioned for example, 1, 2 or 3 bases from the terminal mismatch. Thus, for example, in relation to the determination of the presence of a normal homozygote, heterozygote or affected homozygote in respect of the Z allele of the $\alpha$1 antitrypsin gene we have found that good results may be obtained if the third nucleotide from the 3' terminal nucleotide is altered to generate a mismatch in use. Thus for example we have found that the presence of a C instead of an A as the third nucleotide from the 3' terminus of the diagnostic primer enables normal homozygotes, heterozygotes and affected homozygotes in respect of the Z allele to be readily distinguished. The best design of diagnostic primer may thus be determined by straightforward experimentation based on the above criteria, such experimentation being within the ability of the molecular biologist of ordinary skill.

When using ARMS primers having additional destabilising mismatches adjacent to the 3' terminal nucleotide, we unexpectedly found that ARMS primer extension can still occur despite non-complementarity between the 3' terminal nucleotide of the ARMS primer and the suspected variant nucleotide. This is believed to arise due to complementary binding of at least a terminal nucleotide of the diagnostic primer with corresponding nucleotides in the target DNA adjacent the suspected variant nucleotide. Whilst we do not wish to be bound by theoretical considerations, the primer may bend on the target sequence, or vice versa, to form a loop or "elbow".

The above observation lead to the discovery of a method for detecting more than one variant nucleotide in a target base sequence using a single diagnostic primer.

According to a further aspect of the present invention we now provide a method for detecting the presence or absence of at least one variant nucleotide in a target base sequence comprised in a nucleic acid sample, which method comprises treating the target base sequence, together or sequentially with appropriate nucleoside triphosphates, an agent for polymerisation of the nucleoside triphosphates and a diagnostic primer for a diagnostic portion of the target base sequence under hybridising conditions, the nucleotide sequence of the diagnostic primer being such that it is substantially complementary to the diagnostic portion, a terminal nucleotide of the diagnostic primer being either complementary (i) to at least two suspected variant nucleotides or (ii) to at least two corresponding normal nucleotides; whereby an extension product of the diagnostic primer is synthesised when the terminal nucleotide of the diagnostic primer is complementary to at least one corresponding variant or normal nucleotide in the target base sequence; no extension product being synthesised when the terminal nucleotide of the diagnostic primer is non-complementary to all suspected variant nucleotides or non-complementary to all corresponding normal nucleotides in the target base sequence; and detecting the presence or absence of the suspected variant nucleotides from the presence or absence of extension product.

Conveniently, a corresponding amplification primer is used, any extension product of the diagnostic primer being capable of serving as a template for synthesis of an extension product of the amplification primer after separation from its complement.

The above method may be used in combination with any convenient aspect of the ARMS invention as disclosed in our European patent application no. 0332435 or as described herein. We believe that the number of suspected variant nucleotides which may be analysed using a single diagnostic primer is limited only by practical considerations. Conveniently up to five, up to four or up to three suspected variant nucleotides may be analysed. In particular two suspected variant nucleotides are analysed using a single diagnostic primer.

Appropriate diagnostic primer design will depend on the variant nucleotides to be analysed and the distance(s) between them. Convenient distances included up to 20, up to 15, more conveniently up to 10, up to 5 bases, such as up to 3 bases. It will be appreciated that the primer may be designed so that the corresponding "elbow" arises either in the primer or in the target base sequence. If appropriate the "elbow" may be designed to favour internal hybridisation thus providing additional stabilisation, for example using universal bases such as inosine within the "elbow".

It will be appreciated that where the method gives rise to primer extension/amplification products from more than one suspected variant nucleotide it will be necessary to separate these according to size for direct discrimination. Alternatively, the formation of an ARMS product may prompt further individual analysis.

The above method is conveniently used in the diagnosis of CF mutations, for example W1282X and R1283M which are within 3bp of each other. A convenient primer which hybridises to both mutations is shown below:

```
                                            TGAA

                                            |  /

         R1283M target            ATGGA AGC

                                   ||||| |||

         primer sequence      3'TACCTTTCG..........

                                   |x|||||||

         W1282X target          TGAAGGAAGAC.........
```

The primer shown above has been shown to anneal to R1283M and W1282X DNA as shown by the results of ARMS reaction using both these target sequences. The R1283M target is shown looping out from the primer sequence. There is, at present, no evidence that this is where the loop or "elbow" actually occurs.

Further convenient target base sequences comprising adjacent mutations include RAS oncogene sequences (see for example J.L. Boos et al, Nature 327, 293 (1987).

In standard ARMS assays the lengths of the products to be detected may be readily adjusted with reference to the corresponding amplification primers. In overARMS assays of the present invention the products are often of similar size and care is required when distinguishing these. We have now found that primers used in the methods of the present invention are conveniently provided with tail sequences which do not hybridise to the target base sequence(s). As illustrated in Example 7 we have found that a tail sequence may be used to provide a larger primer extension/amplification product whilst essentially retaining the hybridisation characteristics of a corresponding non-tailed primer. Any convenient length of tail may be employed, such as up to 100, 90, 80, 70, 60 or, more conveniently, up to 50, 40, 30 or up to 20 bases.

In addition, we have unexpectedly found that the use of non-homologous tail sequences may also be used to increase the yield of primer extension/amplification product. As illustrated in Example 8 the relative yield of a particular product was enhanced using a GC rich tail.

Multiple amplification products arising from the methods of the present invention may be distinguished by a variety of techniques. Thus, for example, probes may be employed for each suspected amplified product, each probe carrying a different and distinguishable signal or residue capable of producing a signal.

Such signals and residues capable of producing a signal are discussed in detail in our European Patent Publication No. 246,864, but might for example include the solid phase amplification system described by Wang C G in World Biotech Report 1986 vol. 2, part 2 pages 33-37, (Diagnostics Healthcare Proceedings of the conference held in November 1986, San Francisco) in which microbeads formed with many chosen trace elements are conjugated to the probe. The presence of specific probes may be detected by x-ray fluorescent analysis. Such techniques would generally be simple and straightforward to apply since it would only be necessary to detect the existence of an amplification product rather than distinguish between sequences differing by as little as a single nucleotide.

The presence or absence of a given potential variant nucleotide may thus advantageously be detected by electrophoretic techniques, in which the different amplified products obtained may be distributed according to their molecular weight and thereby identified for example by autoradiography or fluorescent techniques. The lengths of the different products may only differ by a single nucleotide, but preferably the lengths will differ by at least 3 nucleotides. The process of the present invention is preferably effected by the use of an intercalating dye such as ethidium bromide which may be visualised as an orange fluorescence upon ultraviolet irradiation. Thus the presence or absence of a plurality of potential variant nucleotides in a single sample may be rapidly, accurately and easily determined. If desired the diagnostic primer(s) and/or the amplification primer(s) may be marked or labelled for example by the use of a fluorophore. Thus, for example, each different diagnostic primer or amplification primer may carry a different fluorophore so that the results of a battery of tests may be read from an electrophoresis gel for example by a laser scanner, thus enabling automation of the method of the present invention. Alternatively the presence or absence of an amplified product may simply be assessed by the use of a solvent capable of selectively dissolving nucleoside triphosphates, but not capable of dissolving a nucleotide sequence (for example DNA). Trichloroacetic acid (TCA) is an example of such a solvent. Thus for example the presence or absence of an amplified product may be determined by TCA precipitation of amplified reaction mixtures. Where incorporation of the appropriate nucleoside triphosphates has occurred in an exponential reaction series then substantially greater amounts of TCA insoluble material will be present than where no extension of the diagnostic primer has occurred. Quantification of insoluble material might be accomplished by known methods. Thus for example the nucleoside triphosphates might be labelled (for example by a radioactive or fluorescent marker), the reaction mixture may be subjected to for example centrifugation, the liquid present decanted off and either the liquid or the insoluble product subjected to appropriate detection techniques such as radioactive counting or fluorescence determination.

According to a further feature of the present invention we provide a set of at least two nucleotide primers of from about 5 to 50 bp for use in the method of the present invention, a terminal nucleotide of each primer being complementary to either a suspected variant nucleotide associated with a known genetic disorder or to the corresponding normal nucleotide, the remainder of the said sequence being substantially complementary to the corresponding target base sequence adjacent the suspected variant nucleotide or corresponding normal nucleotide, the primers being such that when used as diagnostic primers in the method of the present invention an extension product of a diagnostic primer is synthesised when the terminal nucleotide of the diagnostic primer is complementary to the corresponding nucleotide in the target base sequence, no extension product being synthesised when the said terminal nucleotide of the diagnostic primer is not complementary to the corresponding nucleotide in the target base sequence and wherein the potential extension products of diagnostic primers for more than one diagnostic portion of the target base sequence comprise a complementary overlap.

According to a still further feature of the present invention we provide a primer for use in the present invention, the primer being substantially complementary to a diagnostic region of a target base sequence, a terminal nucleotide of the primer being either complementary (i) to at least two suspected variant nucleotides or (ii) to at least two corresponding normal nucleotides; the primer being such that when used as a diagnostic primer in the invention an extension product of the diagnostic primer is synthesised when the terminal nucleotide of the diagnostic primer is complementary to at least one corresponding variant or normal nucleotide in the diagnostic region of the target base sequence; no extension product being synthesised when the terminal nucleotide of the diagnostic primer is either non-complementary to all variant nucleotides or non-complementary to all corresponding normal nucleotides in the target base sequence.

Conveniently the terminal nucleotide being complementary to either a suspected variant nucleotide or to the corresponding normal nucleotide is at the 3′ end of the nucleotide sequence. Preferably the suspected variant nucleotide results from a point mutation of the corresponding normal sequence.

According to four further features of the present invention we provide nucleotide sequences as defined immediately above and wherein a terminal nucleotide of the nucleotide sequence is complementary to a variant nucleotide resulting from a change of the corresponding normal nucleotide to (i) A, (ii) G, (iii) C, (iv) T or U respectively.

The invention will now be illustrated but not limited with reference to the following Examples, Figures and Tables wherein:-

**Figure 1** is a summary diagram of the Cystic Fibrosis (CF) gene indicating the location of the exons, the mutations described in the text and the location and orientation of the ARMS primers.

**Figure 2** shows the results of an ARMS analysis using the F508/I507 specific primers 1710 and 1711. For each sample analysed there are two reactions and these are adjacent on the gel photograph. The first track contains the products of the normal reaction and the second the products of the mutant reaction. Both reactions contain the control α1antitrypsin primers and the products of these primers are labelled "c"; the products of ARMS primers are labelled F508. The track labelled "M" contains molecular weight markers. The results of the ARMS analyses are:

Sample   1 F508 heterozygote

        2 F508 heterozygote

        3 F508 heterozygote

        4 F508 homozygous mutant

        5 normal

        c no DNA control

        6 normal

        7 normal

        8 normal

**Figure 3** illustrates how two sets of ARMS primers can be used to identify and discriminate the F508 and I507 mutations.

F508N        = F508 Normal primer (1880)

F508M      = F508 Mutant primer (1879)

508/507N    = F508/I507 Normal primer (1878)

508/507M   = F508/I507 Mutant primer (1877)

Primer sequences are indicated in Table 1. Only a small section of the normal (a), mutant F508 (b) and mutant I507 (c) CF gene sequences are shown. The two sets of ARMS primers are shown aligned with the CF gene and mismatches are indicated with bold type. The arrows → indicate which combinations of primers and gene sequences give rise to ARMS products.

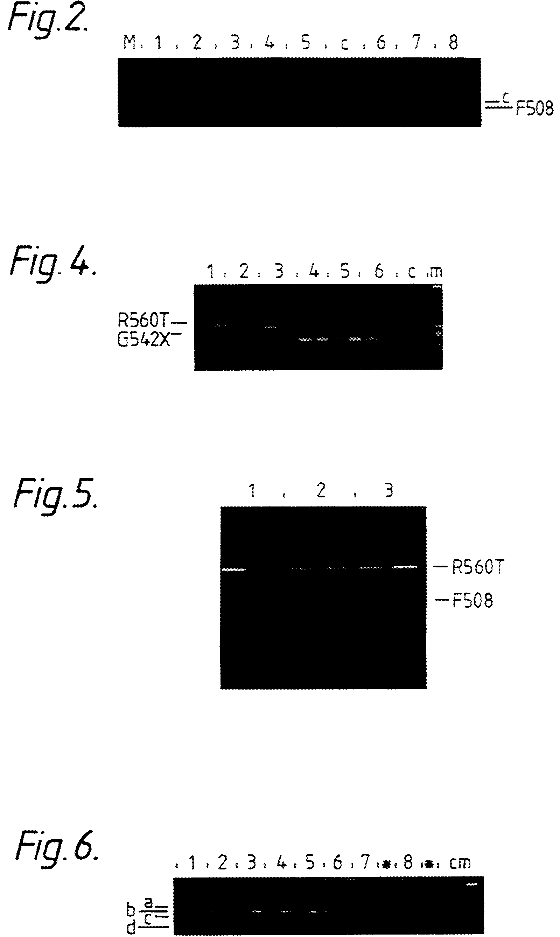

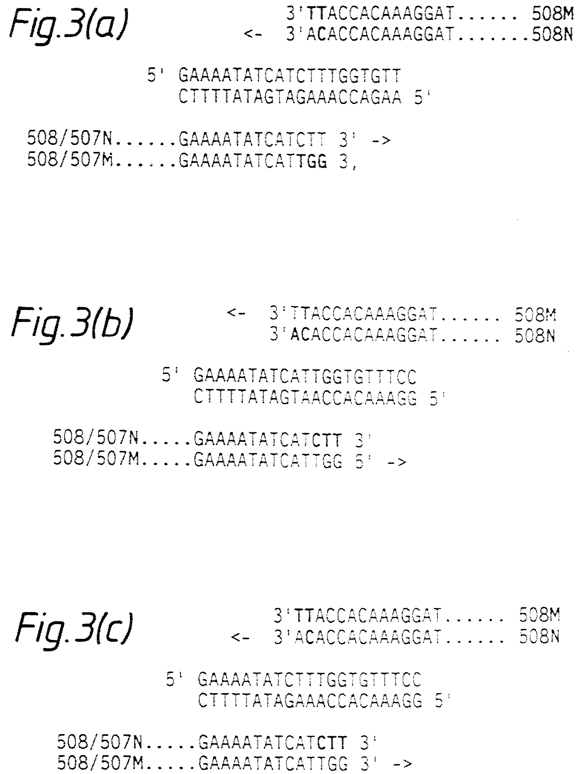

**Figure 4** shows two sets of single ARMS analyses. The first three pairs of tracks are from three samples analysed with ARMS primers specific for the R560T mutation and the second three pairs of tracks are from three samples analysed with primers specific for G542X. In each case the products of the normal ARMS reactions are in the left hand track. Also shown are a negative control sample (c) and molecular weight markers (m). The results of the ARMS analyses are:

Sample   1 - R560T heterozygote

        2 - R560T heterozygote

        3 - normal

        4 - G542X heterozygote

        5 - G542X heterozygote

        6 - normal

**Figure 5** illustrates an ARMS multiplex example combining primers for F508 and R560T. The first track in each pair of samples contains the normal primers and the second contains the mutant primers. The results of the ARMS analyses are:

Sample   1 - F508 heterozygote

        2 - R560T heterozygote; F508 heterozygote

        3 - R560T heterozygote;

**Figure 6** shows the results of an ARMS multiplex combining primers for N1303K(a), G551D(b), R117H(c) and F508 (d). The location of the four ARMS products is indicated on the left hand side of the figure. The first track in each pair contains the normal ARMS primers and the second contains the mutant ARMS primers. The no DNA control and molecular weight markers are indicated by "c" and "m". The results of the analyses are:

Sample   1 - F508,R117H compound heterozygote

2 - F508 homozygous mutant

3 - G551D homozygous mutant

4 - G551D,F508 compound heterozygote

5 - N1303K,G551D compound heterozygote

6 - N1303K,F508 compound heterozygote

7 - N1303K,F508 compound heterozygote

8 - normal

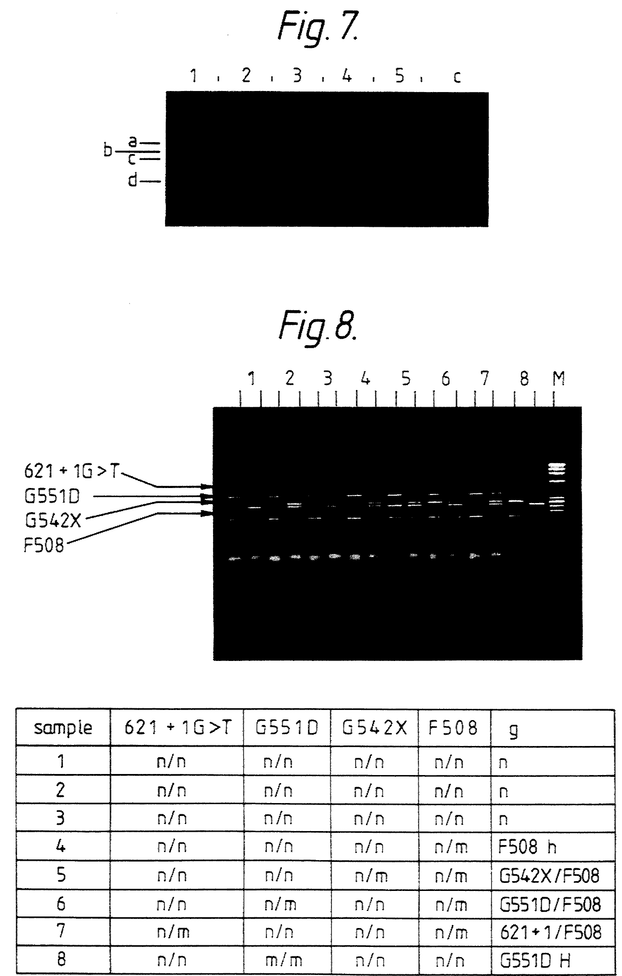

**Figure 7** shows the results of an ARMS multiplex combining primers for N1303K(a), G551D(b), R117H(c) and F508(d). The location of the four ARMS products is indicated on the left hand side of the figure. The first track in each pair contains the normal F508 and G551D primers and the mutant N1303K and R117H primers. The complementary primers are in the second track in each pair. The no DNA control track is indicated -"c". The results of the analyses are:

Sample 1 - F508,G551D compound heterozygote

2 - F508 heterozygote

3 - F508,N1303K compound heterozygote

4 - G551D heterozygote

5 - normal

**Figure 8** demonstrates an overARMS multiplex analysis. The location of the products of the four ARMS reactions combined in the multiplex (621+G>T, G551D, G542X and F508) is indicated on the figure together with the results of the analysis. Abbreviations used are n = normal; m = mutant; g = genotype; h = heterozygote and H = homozygote.

**Figure 9** illustrates convenient arrangements for diagnostic primers used in the method of the invention. In (i), (iii) and (iv) the primers (DP1-DP3) are provided on the same target base sequence. In (iii) and (iv) the primers comprise a complementary overlap. In (ii) the primers comprise a complementary overlap but are on different target base sequences.

**Figure 10** illustrates diagnostic primers (DP1-DP3) used in combination with corresponding amplification primer(s) (AP1-AP3).

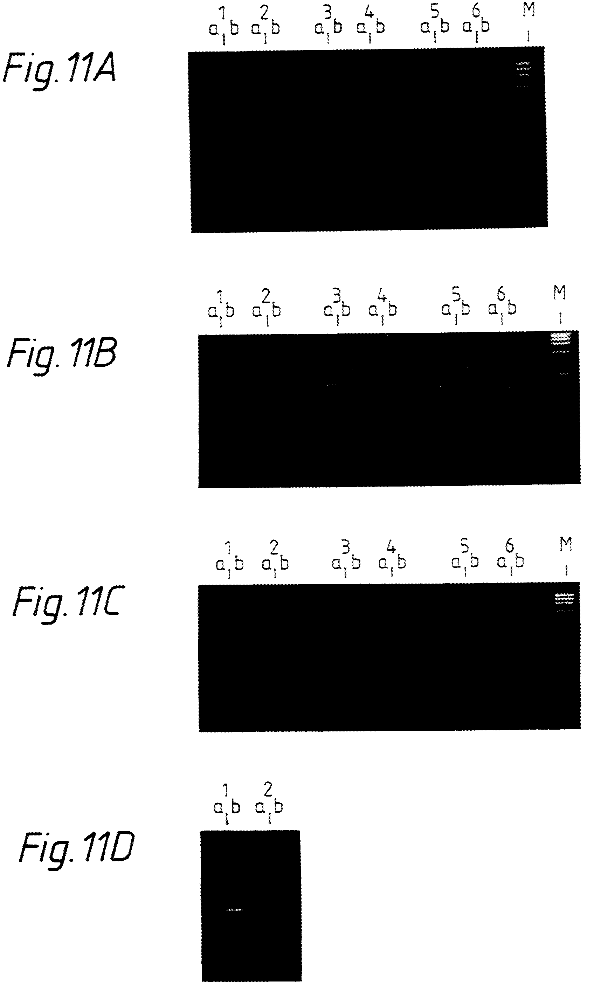

**Figure 11** shows eleven sets of single ARMS analyses. For each ARMS test there are two samples. The first sample in each pair is from a normal individual, the second is from an individual heterozygous for the mutation under test. All of the tests, except for N1303K, contain an additional control reaction. For each sample the lower case a indicates the track containing the products of the normal reaction and the lower case b indicates the track containing the products of the mutant reaction. M indicates a marker track.

The ARMS tests are arranged as follows:-

A,    Samples 1 and 2 - R560T ARMS test

Samples 3 and 4 - G551D ARMS test

Samples 5 and 6 - G542X ARMS test

B,    Samples 1 and 2 - R117H ARMS test

Samples 3 and 4 - 1717G>A ARMS test

Samples 5 and 6 - W1282X ARMS test

C,    Samples 1 and 2 - R553X ARMS test

Samples 3 and 4 - 621+1G>T ARMS test

Samples 5 and 6 - N1303K ARMS test

D,    Samples 1 and 2 - G85E ARMS test

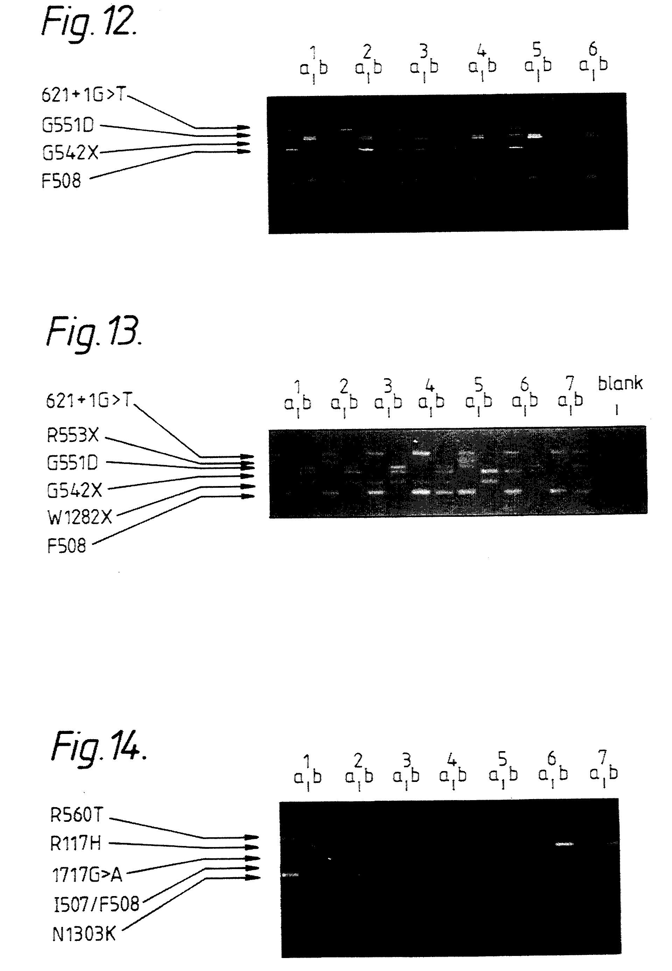

**Figure 12** showes the results of an ARMS multiplex analysis using the revised standard test. The location of the products of the four ARMS reactions combined in the multiplex (621+1G>T, G551D, G542X and F508) is indicated on the figure. The first track in each pair, labelled a, contains the normal 621+1G>T and F508 primers and the mutant G551D and G542X primers. The complementary primers are in the second track in each pair, labelled b.

The results of the analyses are:

Sample 1 - normal

2 - F508 homozygote

3 - F508 heterozygote

4 - G542X heterozygote

5 - G551D heterozygote

6 - 621+1G>T heterozygote

**Figure 13** shows the results of an ARMS mutiplex analysis using the Standard plus test. The location of the products of the seven ARMS reactions combined in the multiplex (621+1G>T, R553X, G551D, G542X, W1282X/R1283M and F508) is indicated on the figure.

The first track labelled a in each pair contains the normal 621+1 and F508 primers and the mutant R5523X, G551D, G542X and W1282X/R1283M primers. The complementary primers are in the second track labelled b in each pair.

The results of the analyses are:

Sample 1 - normal

2 - R553 heterozygote

3 - W1282X heterozygote

4 - R1283M, F508 compound heterozygote

5 - R553X, G551D compound heterozygote

6 - G542X heterozygote

7 - 621+1G>T, F508 compound heterozygote

**Figure 14** shows the results of an ARMS multiplex analysis using the extended mix. The location of the products of the five ARMS reactions combined in the multiplex (R560T, R117H, 1717-1G>A, DI507/DF508 and N1303K) is indicated on the figure.

The first track labelled a in each pair contains the normal R560T, 1717-1G>A and N1303K primers and the mutant R117H and DI507/DF508 primers. The complementary primers are in the second track labelled b in each pair.

The results of the analyses are:

Sample 1 - normal

2 - N1303K, DI507/DF508 compound heterozygote

3 - 1717-1G>A, DI507/DF508 compound heterozygote

4 - R117H, DI507/DF508 compound heterozygote

5 - R560T, DI507/DF508 compound heterozygote

6 - DI507/DF508 homozygote

7 - DI507/DF508 heterozygote

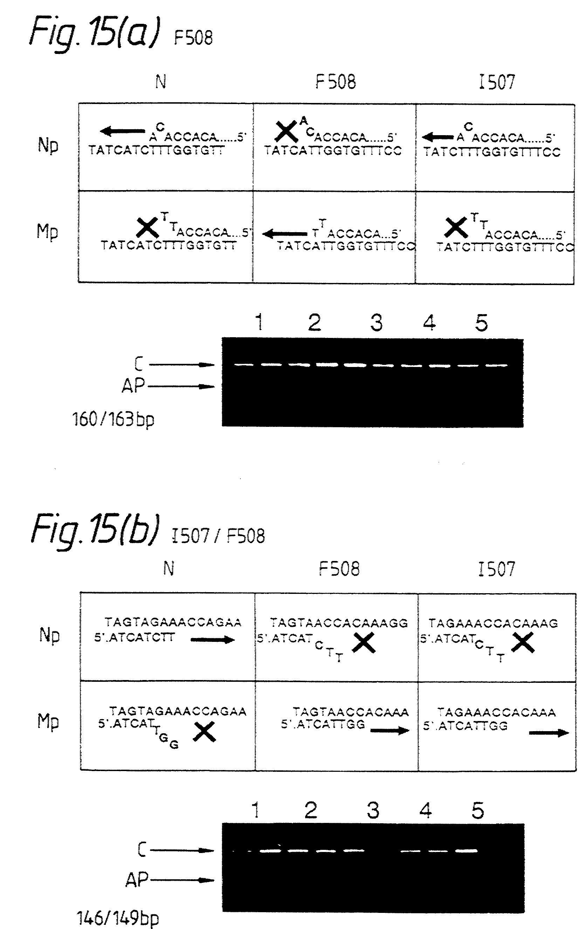

**Figure 15** illustrates further specific ARMS tests. In a) the test is for F508. The panel indicates the sequences of the ARMS primers and the target DNA. The diagrams in the boxes align the normal and mutant ARMS primers (3′-5′) with the normal / F508 and / I507 target DNA sequences. Complementary bases in the ARMS primers are shown in normal type whereas non-complementary bases are shown in bold type and displaced from the target sequence. An arrow indicates primer/target combinations which act as a substrate for Taq DNA polymerase under standard ARMS conditions and a cross shows where extension does not occur. Below the boxes are the results of an ARMS analysis using normal primer 1880 and mutant primer 1879 (see Table 1). For each of the five samples (1-5) there are two tracks, the first shows the products from the normal ARMS reaction and the second from the corresponding mutant reaction. Abbreviations used are N = normal; Np = normal primer; M = mutant; Mp = mutant primer; C = control; AP = ARMS product. In b) the test is for I507/508. The panel shows the corresponding information for the / F508, / I507 specific primers (1878 and 1877, see Table 1). The ARMS primer sequence is written 5′-3′. The genotypes of the five samples are 1) normal, 2) / I507 heterozygote, 3) / F508 homozygote, 4) / F507 heterozygote and 5) / F508, / I507 compound heterozygote. These genotypes can be deduced from the combined results of both analyses.

**Table 1** lists the complete set of primers used in the study. For each CF mutation all the normal (N), mutant (M) and common (C) ARMS primers described in the text are listed. For CF point mutations, any additional mismatches introduced into the normal and mutant primers are shown. If the additional mismatch is not at the penultimate base then this is indicated in the Table. The nucleotide sequences of all of the primers are listed.

**Table 2** shows the results obtained in the first overARMS attempt described in the text. For the 8 samples tested, the genotype is listed followed by the results obtained with the three pairs of ARMS primers. The presence of a + indicates that product was observed.

**Table 3** summarises the primers used in the overARMS multiplex described in Example 3. The numbers are the primer reference numbers described in Table 1. The A-mix and B-mix refers to the two reactions of the ARMS test.

**Table 4** lists observed mutations of the cystic fibrosis (CF) gene.

**Table 5** indicates primers used in the revised standard overARMS multiplex (Example 6).

**Table 6** indicates primers used in the standard plus overARMS multiplex (Example 7).

In the Examples below the following materials and methods were employed:

Preparation of genomic DNA

Human genomic DNA samples were prepared by one of three methods:-

a) standard extraction from blood cells using the method of Kunkel L.M., Smith K.D and Boyer S.H., 1977, Proc. Natl. Acad. Sci. USA, 74, 1245-49.

b) rapid extraction from peripheral blood cells. Each 200µl aliquot of previously frozen blood was diluted with 800µl of freshly made 170mM NH4Cl solution. After agitating gently for 20 min, the white cells were collected by centrifugation in a microfuge for 2 min. The white cell pellet was washed four times with 300µl of 10mM NaCl/10mM EDTA, the cells being collected by centrifugation for 15 sec between washes. After washing, the white cell pellet was resuspended in 500µl of 50mM NaOH and vortexed for 15 sec. The cell suspension was placed in a boiling water bath for 20 min and neutralised at room temperature by the addition of 100µl of 1M Tris.Cl (pH 7.5). After vortexing briefly, the remaining cellular debris was pelleted in a microfuge for 10 sec. 5µl of DNA prepared in this way was used in ARMS reactions with the standard mix.

c) rapid extraction from buccal epithelial cells. Buccal epithelial cells were collected from donors using either the mouthwash or buccal swab methods. When the mouthwash method was used, donors provided cells in 10 mls of 4% sucrose after agitating in their mouths for a minimum of 20 sec. After centrifugation at 2000 rpm for 10 min, the supernatant was discarded and the cells resuspended in 500µl of 10mM NaCl/10mM EDTA and transferred to screw-topped microfuge tubes. The cells were again pelleted in a microfuge for 15 sec and resuspended in 500µl of 50mM NaOH as above. The remainder of the procedure was then exactly as has been described for blood cells in section b). For buccal swabs, cotton buds were saturated with 4% sucrose. The donors cheek was swabbed briefly (5 sec) and the head of the cotton bud added to 500µl of 170mM NH4Cl in a screw-topped microfuge tube. After vortexing for 15 sec, the bud was removed from the tube and the epithelial cells collected by microfugation for 15 sec. After removal of the supernatant, the cells were washed with 300µl of 10mM NaCl/ 10mM EDTA and collected by microfuging for 15 sec. The cells were resuspended in 500yl of 50mM NaOH and the remainder of the procedure detailed for blood in section b) was followed.

ARMS reaction conditions

For ARMS reactions, standard conditions were 1x ARMS buffer (50mM KCl, 10mM Tris.Cl pH8.3, 1.2mM MgCl2, 0.1% gelatin) 100µM dATP, dCTP, dGTP, TTP and the appropriate individual ARMS primers at 1µM. 40µl aliquots of the appropriate reaction mixes were used in 0.5ml polypropylene tube. 5µl (~25ng) of human genomic DNA and 1 unit (2µl) of Taq polymerase (Cetus-Amplitaq) were used in each individual ARMS reaction. Each ARMS reaction was overlayed with one drop of mineral oil. The procedure used was as follows:-

The reactions containing all components except enzyme were heated at 94°C for 5 min. After the addition of enzyme, 35 cycles of PCR were performed under the following conditions: 94°C denaturation for 2 min; 60°C annealing for 2 min; 72°C extension for 2 min. The procedure was completed by the extension of the last 72°C incubation by a further 10 min.

Analysis of PCR products was by gel electrophoresis through 3% Nu-sieve agarose (2:1) containing ethidium bromide.

### Example 1

**ARMS analysis of single CF mutations**

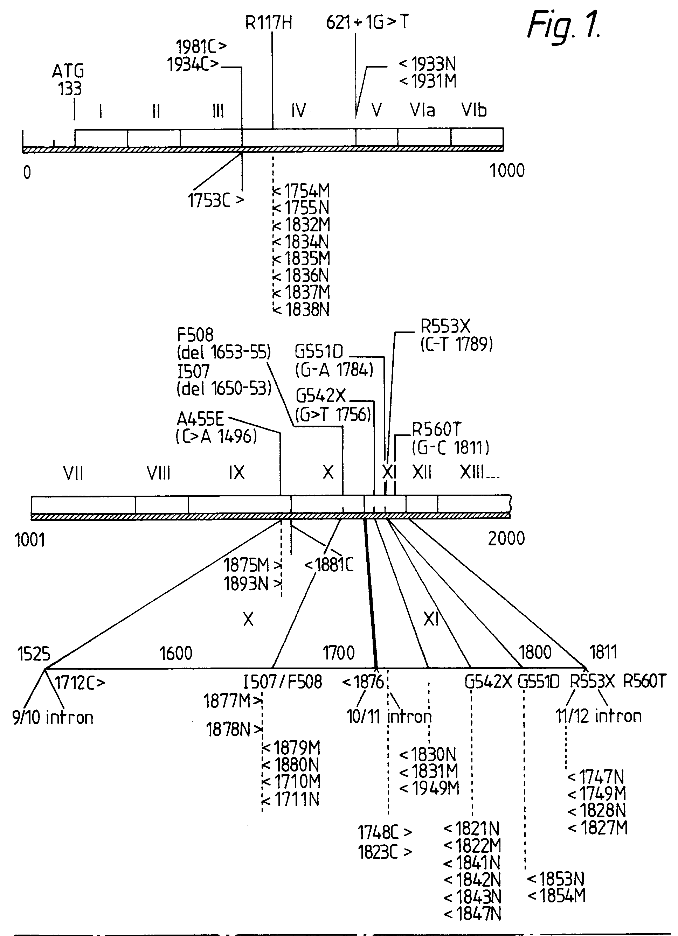

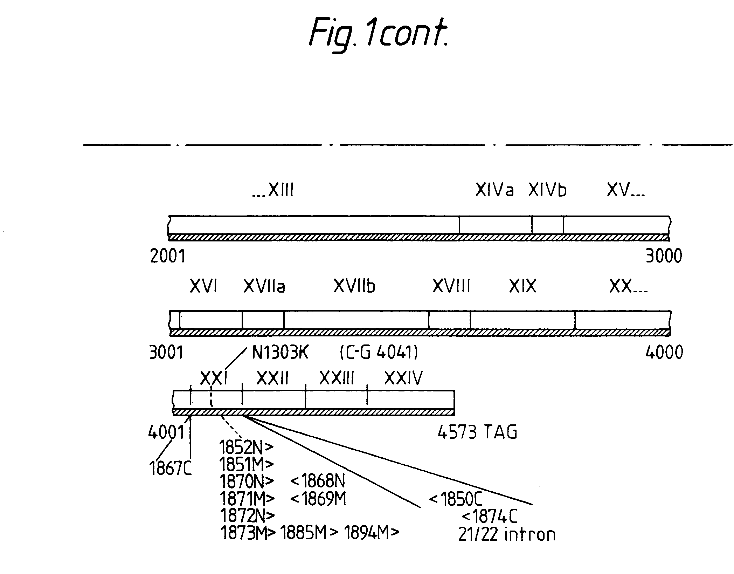

ARMS tests were initially developed to allow the identification of single CF mutations. Reactions were developed for the nine CF mutations shown in Table 1. A map of the CF gene, showing the location of the CF mutations and the ARMS primers is given in in Figure 1.

The format of these single tests was similar to that described by C.R. Newton et al (Nucleic Acids Research, 1989, 17, (7), 2503-2516) in that additional control primers were included in the tests to ensure correct performance of the PCR reactions. The control primers (667 and 668 - see Table 1) amplified a 220 bp fragment of the α-1-anti trypsin gene. The tests were developed using human genomic DNA prepared by the standard method (see above) and, except where indicated in the text, the ARMS reaction conditions were as described in the Materials and Methods section.

It should be noted that for some of the mutations described below DNA samples from homozygous mutant individuals were not available therefore, for these mutations, it has not been possible to test the specificity of the normal ARMS primers. This is the case for I507, R560T, R117H, N1303K, R553X and 621+1G>T.

**1+2) F508 and I507**

An ARMS test for these mutations has alredy been described by C.R. Newton et al (Lancet, 1990, 335, (8699), 1217-1219). In the CF gene the I507 and F508 mutations are adajacent (Kerem B., Zielenski J., Markiewicz D., Bozon D., Gazit E., Yahaf J., Kennedy D., Riordan J.R., Collins F.S., Rommens J.R. and Tsui L.-C, Proc. Natl. Acad. Sci. USA, 1990, 87, 8447) and the published ARMS primers identify both of them. These original primers (1710 and 1711) are listed in Table 1 and a typical analysis is shown in Figure 2.

To allow the differential diagnosis of F508 and I507 two further ARMS test were developed. The first was similar to the original test in that both mutations were identified simultaneously (primers 1879 and 1880 - Table 1). The second ARMS test was designed such that genes carrying F508 would be identified as mutant whereas I507 genes would appear normal (primers 1877 and 1878). These primers are listed in Table 1 and a diagram illustrating their specificity is shown in Fig 3. It is clear that, by sequential use of these ARMS tests, I507 and F508 individuals can be differentiated.

### 3) R560T

The R560T mutation is the result of a G to C change at bp 1811 in exon 11 of the CF gene (Kerem et al, 1990). ARMS primers 1747 and 1749 were designed to allow the diagnosis of this mutation. In addition to the 3′ terminal mismatch caused by the presence or absence of the R560T mutation, the introduction of an adenine residue at the penultimate base of both the normal and mutant ARMS primers gave an additional G/A mismatch. The purpose of this additional mismatch was to improve the specificity of the reaction. Details of the primers are given in Table 1 and a typical analysis is shown in Figure 4.

### 4) R117H

This mutation is caused by a G to A change at bp 482 in exon 4 (M. Dean et al, Cell, 1990, 61, 863-870). ARMS primers for the normal and mutant alleles were designed with an additional mismatch included at the penultimate base. In the first attempt to design an R117H ARMS test the primers used were numbers 1836 (N), 1835 (M) and 1753 (C) (see Table 1) which included an additional C/A mismatch. When these primers were tested in an ARMS reaction with DNA from normal individuals or from individuals carrying the R117H mutation, it was found that the test was not completely specific. Although the DNA from R117H individuals gave a strong signal with the mutant ARMS primer (1835) there was also a signal, albeit weaker, when normal DNA was used.

Three approaches were used to improve the specificity of the ARMS test:

(i) It was found that reducing the concentration of the ARMS primers from 1.0μM to 0.125μM gave the desired specificity although there was also a reduction in the overall strength of the signal.

(ii) In the initial attempt using primers 1835 and 1836, the α1AT control primers had not been included. When these control primers were included in the ARMS reaction the desired specificity was then achieved.

(iii) It was also possible to increases the specificity of the ARMS reaction by redesigning the primers to increase the disruption caused by the additional mismatch. Two further pairs of ARMS primers were synthesised; in these the additional mismatch was changed from the original C/A to a C/T (Primer 1834 and 1837) or to a C/C (1838 and 1832). When these primers were used in ARMS reactions, without the internal control primers, it was found that the C/T mismatch gave some improvement in specificity and the C/C mismatch gave the required specificity ie. product was not observed in the mutant reaction when DNA from normal indivuals was tested.

### 5) G542X

The cause of this mutation is a G>T change at bp 1756 (Kerem et al, 1990). ARMS primers were designed with an additional G/A mismatch at the penultimate base. When these primers (1830-N, 1831-M and 1823-C see Table 1) were used in an ARMS test in combination with the α1At control primers the normal and mutant reactions were specific. A typical analysis is shown in Figure 4.

### 6) R553X

An ARMS test for this mutation (a C>T change at bp 1789 (G.R. Cutting et al, Nature, 1990, 346, 366-368) was designed such that an additional G/A mismatch would be formed with both the normal and mutant primers. When these primers (1853, 1854 and 1823; see Table 1) were used in a standard ARMS test along with the α1AT control primers the desired specificity was obtained.

### 7) G551D

The intial attempt to design an ARMS test for the G551D mutation (a G>A change at bp 1784; Cutting et al, Nature, 1990, 346, 366-368) was not successful due to a lack of specificity in both the normal and mutant ARMS reactions. The primers used (1822-M, 1821-N and 1823-C; see Table 1) were designed to give an additional T/C mismatch at the penultimate base.

The non-specific binding observed with the mutant ARMS primers, ie. product in the normal reaction was slight and was not observed when the $\alpha$1At control primers were included in the reaction. In the case of the normal primers however, a significant yield of normal product was obtained when DNA from an individual homozygous for G551D was analysed. Three approaches were used to solve this problem.

As with the development of the R117H ARMS test the approaches used to increase the specificity were; inclusion of $\alpha$1aT control primers; reduction of the primer concentration and redesign of the ARMS primers to increase the level of destablisation caused by the additional mismatch. All of these approaches were successful. When the normal primer concentration was reduced from 1.0$\mu$M to 0.25$\mu$M specificity was improved although there was a slight compromise with product yield.

Four additional normal primers were designed. The additional mismatch and specificity is shown below:
primer 1847, C/T mismatch at -3, no improvement of specificity;
primer 1841, C/T at -3, G/T at -2, slight improvement of specificity;
primer 1842, C/T at -3, C/T at -2, specific; significantly reduced yield;
primer 1843, C/A at -3, C/T at -2, specific; slightly reduced yield

Of the four addtional primers 1843 was selected as the preferred candidate for a single mutation ARMS test.

### 8) N1303K

The cause of this mutation is a C>G change at bp 4041 in exon 21 of the CF gene. The initial attempt to develop an ARMS test for this mutation was not successful. The primers which were designed for this test (1852, 1851 and 1850 - see Table 1) were specific for the mutation but the product yield was very low and the ARMS products were barely visible. The extra mismatch introduced into the ARMS primers 1852 and 1851 was a C/T at the penultimate base. The sequence around the N1303K mutation has a low G+C content (2/10) and it seemed likely that this coupled with the mismatch may have prevented efficient annealing of the ARMS primers. This problem was tackled in two ways. In the first approach the severity of the mismatch was reduced such that in the ARMS primer pair 1870/1871 a weak G/T additional mismatch was included and in the primer pair 1873/1872 no additional mismatch was included. A second approach was to switch the direction of the ARMS reaction such that the ARMS primer now annealed to the opposite side of the N1303K mutation. The G+C content at this side was 6/10 and an additional C/T mismatch was included at the penultimate base.

Both of these approaches were shown to work. Using the first method the primer pair without additional mismatches gave the desired specificity and improved product yield and the second method was also successful giving higher product yields and maintaining specificity.

### 9) 621+1 G>T

This mutation is a change to the first base in intron 4 of the CF gene and probably interferes with splicing (B. Kerem et al, Proc. Natl. Acad. Sci. USA, 1990, 87, 8447). ARMS primers for this mutation (1931, 1933 and 1934 - see Table 1) were designed to give an additional C/T mismatch at the penultimate base. When these primers were tested with DNA from normal individuals and 621+1 heterozygotes the desired specificity and product yields were obtained.

### Example 2

### Multiplex ARMS Analysis of several CF mutations

To improve the usefulness of the CF ARMS tests and to reduce the time and effort involved in the analysis of several CF mutations we wished to combine several ARMS reactions to produce a multiplex ARMS analysis. The initial attempt, described below, involved F508 and R560T. Following on from, this four ARMS tests were selected to be multiplexed together; these were F508, R117H, G551D and N1303K. During the development of the single ARMS tests the size of the ARMS products had been adjusted to ensure that these four mutations would be distinguishable upon analysis. A stepwise approach to the development of this multiplex was adopted which is outlined below. Earlier data from the development of single ARMS tests suggested that the incorporation of a second PCR reaction in the ARMS test ie the α1AT internal control, could affect the specificity and yield of ARMS product. It seemed possible therefore that the optimum primer sequence for a single ARMS test may not function well in a multiplex analysis.

### 1) F508 and R560T

The primers which had previously been shown to work well in the single ARMS tests were combined to give a double test (primers 1710, 1711, 1712, 1747, 1749 and 1748). The concentrations of the individual primers were not altered and all other reaction conditions were kept the same. The results of a typical analysis are shown in Figure 5. It can be seen that the test was able to distinguish both F508 and R560T individuals from normals. There was no interaction between the ARMS primers to produce non-specific products and further optimisation was not required.

### 2) F508 and R117H

As in the above example, when the primers developed for the single ARMS tests were combined without altering their concentrations or reaction conditions the resulting double ARMS test functioned correctly as judged by correct diagnosis of normal and mutant individuals.

### 3) F508 and G551D

In the previous section there were several attempts to obtain a functional G551D single test. When the primers developed in this test (1822-M, 1843-N and 1821-C) were combined with the F508 primers and used in ARMS reactions, there was a significant reduction in the level of G551D normal product observed although the yield of mutant product appeared unchanged. Correct diagnosis was possible using this combination of primers

### 4) F508, G551D and R117H

Following on from the previous examples the three primer sets for the single F508, R117H and G551D tests were combined to form a multiplex. When this ARMS reaction was tested using DNA from normal and mutant individuals a problem was observed in that the G551D normal product was not detectable. Two approaches were taken to solve this problem. In the first the concentration of G551D normal primer (1843) was increased 2 fold or 4 fold - this was partially successful in that the G551D normal product was now visible but the intensity was still reduced relative to other bands. The second approach involved the use of an alternative normal ARMS primer. In the development of the single ARMS test the original normal primer (1821) gave a strong signal on normal DNA but suffered from the problem of non-specific binding in that a faint product band was seen with an individual homozygous for the G551D mutation. This normal primer was tested in the multiplex reaction in place of the 1843 normal primer. The results indicated that the 1821 primer, which was not suitable for a single ARMS test, was ideal for this multiplex. The yield of the normal product was not reduced and the prblem of non-specific binding was not observed in the multiplex reaction. We therefore believe that there is some interaction between primer sets in the ARMS multiplex and this should be taken into account when developing these methods.

### 5) F508, G551D, R117H and N1303K

As described in the previous section there were two functional primer sets for N1303K. One of these (1872, 1874 and 1873) was initially selected and as the N1303K product had been rather faint in the single test it was included here at double concentration (2μM). The results, shown in Figure 6, indicate that this multiplex reaction works satisfactorily. To improve the reliability of the test two changes were introduced: the N1303K primer set was changed to the alternative set described in the previous section (1868, 1869 and 1867), this had the effect of increasing the intensity of the N1303K product without the need for increased primer concentration. The second change involved the format of the test: rather than combine all the normal primers in one ARMS reaction and all the mutant primers in a second it is possible to combine the normal and mutant primers (from different primer sets) in the same reaction. The advantage of this approach is that it is no longer necessary to include an internal control to ensure that the PCR reaction has functioned correctly. Both of these changes were incorporated in the multiplex reaction and an example of a typical analysis is shown in Figure 7.

### Example 3

### Overlapping ARMS (overARMS)

In the previous example the value of a multiplex ARMS technique was demonstrated using four mutations which are well separated in genomic DNA. In the analysis of genomic DNA the situation can also occur where it would be advantageous to be able to detect closely linked mutations. A good example of this is in the cystic fibrosis (CF) gene where there is a cluster of important mutations in exon 11 (G542X, G551D, R553X and R560T) within 55bp of each other. In particular, G542X and G551D are the two most common CF mutations after F508.

In Example 1 single ARMS tests were described for all four of these mutations. It was initially believed that simultaneous diagnosis of these closely linked mutations in a multiplex ARMS reaction would not be successful not least due to interference between homologous amplification products. As mentioned earlier above the present invention is based on the discovery that mulitplex ARMS may be successfuly performed where diagnostic primer extension products of more than one diagnostic portion of the target base sequence comprise a complementary overlap. This unexpected improvement to multiplex ARMS is conveniently referred to as overlapping ARMS (overARMS).

Two examples of overARMS analyses are given below. The first is a preliminary attempt which demonstrates the feasibility of the technique and the second combines overARMS methodology with standard multiplex techniques to produce a robust and reliable test for four of the most common CF mutations.

### 1) G542X, R553X and R560T

In the first section single tests were developed for these mutations. The three pairs of normal and mutant ARMS primers from these single tests (1830, 1831, 1853, 1854, 1747 and 1749) were combined with one of the common primers (1748). The latter was included at a concentration of 3.0µM. ARMS tests using this combination were performed on DNA samples from normal individuals and from individuals carrying combinations of the three mutations. A list of the genotypes of the samples tested and of the results obtained using the overARMS reaction is given in Table 2. It can be seen that in the diagnosis of R560T and G542X the analysis was succesful, furthermore R553X mutant bands can be clearly identified. There was a problem however in that the R553X normal band was not always visible. In a separate experiment involving R553X in a standard multiplex reaction the normal band was also barely visible and it seems likely that by redesigning the normal primer or increasing its concentration that this problem could be overcome. Notwithstanding this problem, the experiment clearly demonstrated the feasibility of the overARMS technique.

### 2) F508, G542X, G551D and 621+1G>T

These are four of the most common CF mutations and a simultaneous test would clearly be a valuable tool to aid in the rapid diagnosis of CF carrier status. The overARMS system of the present invention now facilitates such diagnosis.

Initially the primers identified as suitable for G542X and 621+1G>T in single ARMS tests and F508 and G551D in multiplex ARMS tests were combined into a single ARMS test. The format of the test was such that in one ARMS reaction (A) were the F508 and 621+1 normal primers and the G542X and G551D mutant primers whereas the second reaction contained the remaining primers. The single common primer for the overARMS part of the multiplex was included at double concentration. The first attempt using this combination was successful although the yield of products was not equal thus making interpretation more difficult.

The specific problems and modifications to rectify them are listed below -

The yields of both normal and mutant 621+1G>T products were higher than the other products of the multiplex. The concentration of this primer set was reduced to 0.5µM.

The G551D normal product was less intense than the G542X normal product. This effect was more pronounced when a 621+1G>T individual was tested. Reducing the 621+1 primer concentration leads to some improvement. An alternative G551D normal primer was tested1821. This primer increased the yield of G551D product but reduced the yield of the G542X band. The solution adopted was to double the concentration of the G551D normal primer, the concentration of the common primer was not altered.

The yield of G542X mutant product was low. The mutant ARMS primer was redesigned to reduce the disruption caused by the additional mismatch at the penultimate base and the resulting primer -1949- gave the desired result.

The yield of F508 mutant product was slightly low. The concentration of primer was doubled to overcome this.

The complete set of primers used in this overARMS multiplex are summarised in Table 3. A typical result using the test is shown in Figure 8. It can be clearly seen that the overARMS technique performs well and is suited for a variety of analytical purposes such as the diagnosis of inherited or acquired genetic disease.

## Example 4

**Further development of overARMS.**

(a) The standard overARMS mix described in the previous Example comprises ARMS primers specific for the detection of the following commom mutations of the CF chromosome - Del F 508, 621+1 G>T, G551D and G542X. Individuals carrying all possible combinations of G542X and G551D were tested with the standard mix as described. Individuals affected with the following combinations of mutations were successfully identified:-

(i) G542X normal, G551D normal

(ii) G542X normal, G551D heterozygous mutant

(iii) G542X normal, G551D homozygous mutant

(iv) G551D normal, G542X heterozygous mutant

(v) G551D heterozygous mutant, G542X heterozygous mutant

(vi) G551D normal, G542X homozygous mutant

(b) The overARMS detection method has been tested as part of the standard mix with DNA isolated from a variety of sources of tissue using two types of DNA preparation method. DNA has typically been isolated from white blood cells using the method of Kunkel et al, Proc. Natl. Acad. Sci. USA, 1977, 74, 1245-49. Alternatively, DNA can be fractionated from the white cells purified from 200µl of blood by boiling dilute sodium hydroxide as described in the Materials and Methods section. DNA has also been isolated from buccal epithelial cells purified from either mouth swab or mouth rinse samples using an adaptation of this method. The overARMS method has been demonstrated to work effectively when genomic DNA is prepared using any of the preparation methods described above.

(c) The effect of changing the amount of Taq polymerase in the overARMS reactions:

Typically, 1 unit of Taq polymerase is used in each individual standard overARMS reaction. The effect of increasing and decreasing the amount of enzyme has been determined. The standard mix was tested with 0.1U, 0.5U, 1.0U and 2U of Taq polymerase (Cetus-Amplitaq) under otherwise identical reaction conditions. A panel of six human genomic DNAs carrying various combinations of CF mutations was prepared from white blood cells using the proteinase K method described above. These DNAs were used as substrates for the overARMS PCR to determine if the correct diagnoses were identified. The genotypes of the substrate DNAs used were as follows: normal, dell, G542X/F508, G551D/F508, 621+1 G>T, homozygous G551D. When 0.1U of Taq polymerase was used per reaction tube, no PCR products could be detected by ethidium bromide staining of the electrophoresed reactions after 35 cycles of denaturation, annealing and extension as already described. When the other enzyme amounts were used, the diagnoses of the panel of six genomic DNAs were always determined correctly. The intensity of all PCR product bands stained by ethidium bromide was increased when greater amounts of enzyme were added to the reactions. Further, the relative intensities of the bands representing normal alleles at the sites of possible exon 11 mutations (ie G551D and G542X) could be affected by the amount of enzyme used. At 0.5U of Taq polymerase, the shorter product which represents a normal G542 allele is of greater intensity than the normal G551 allele in individuals unaffected by either of these exon 11 mutations. When 1.0U of Taq polymerase is used the bands detected are of equivalent intensity whereas an increase in enzyme amount to the 2U levels renders the longer product containing the normal G551 allele more intense in relation to the normal G542 allele.

(d) The effect of DNA quantity on overARMs PCR fidelity

The panel of 6 human genomic DNAs described in (c) above were used in standard mix overARMS PCR reactions to determine the effect of variation in the quantity of DNA on overARMS diagnosis. 2.5ng, 25ng and 250ng of DNA were evaluated. In all cases, the genotype of the substrate DNA was determined correctly. The DNA quantity was observed to have no effect on the relative intensities of the PCR products formed in the exon 11 region. Increasing the ammount of DNA used caused a general increase in the intensity of overARMS PCR products. When 250ng of DNA is used, a faint background smear is also present in the electrophoresis tracks.

## Example 5

**Additional single ARMS tests**

1) <u>1717-1 G>A</u>

This mutation is a G>A substitution at the acceptor splice of intron 10 of the CF gene. Three sets of ARMS primers were synthesised in which the addtional 3′ mis-match was varied to evaluate the effect of G/T (2065 and 2070), G/G (2067 and 2069) and G/A (2066 and 2068) mis-matches at position -2 on ARMS specificity. These primers were used in combination with the common primer 1823 to form a 220bp product. The mutant primers with G/T and G/G mis-matches were both non-specific, generating faint bands on normal DNA, although the effect was more pronounced with the G/T mis-match. The G/A mis/matched mutant ARMS primer appeared specific. When control primers 677 and 678 were included in the ARMS reactions it was found that the G/G mutant ARMs primers became specific. The G/A mis-matched primer retained speficicity but generated reduced amounts of mutant ARMS product. An ARMS test for 1717-1 G>A is illustrated in Figure 11.

2) <u>W1282X</u>

The W1282X mutations is a G>A substitution at bp 1282 in exon 20 of the CF gene. ARMS primers with either A/C (2010 and 2011), A/G (2013 and 2012) or A/A (2155 and 2109) mis-matches at the penultimate 3′ base were tested for mutant specificity. Mutant ARMS primers generating a G/A mis-match yielded a mutant specific product of 177 bp whilst the A/A and C-A mis-matched primers were non-specific generating faint bands in normal individuals. When the W1282X ARMS tests were performed in combination with either the 677/8 or 1713/4 control reactions 2155 and 2109 primers were specific. 2012 failed to amplify mutant DNA (although the normal reaction was unaffected) and 2011 remained non-specific. An ARMS test for W1282X is illustrated in Fig. 11.

3) <u>G85E</u>

The G85E mutations is the result of a G>A transition at nucleotide 386 in exon 3 of the CF gene. ARMS primers containing an additional C-T mismatch (2248 and 2247) or an additional C-A mismatch (2251 and 2250) were used in combination with common primer 2246 to form a 140 bp product.

In single ARMS reactions, neither mutant primer 2250 nor 2247 were specific for the G85E mutation. When control primers 1713 and 1714 were included in the ARMS reactions at a concentration of 0.5μM a faint non-specific product was still obtained with 2250 but primer 2247 was specific for mutant DNA. An ARMS test for G85E is illustrated in Figure 11.

## Example 6

**Modifications to the Standard ARMS Test**

Data generated in the course of in-house validation (216+ samples) and clinical trial (500+ samples) studies prompted a few minor changes to the Standard ARMS Test described in Table 3.

(i) 621+1 Mutant ARMS Primer (B-Mix)

Follow-up studies to investigate a single discordant result reported by Guy's Hospital, London lead to the discovery a new mutation, 621+3 A>G. It transpired that the 621+3 mutation could also be detected using the 621+1 mutant primer 1931. The 621+3 mutation however is believed to be benign and ideally should not be detected in a carrier screening assay. Consequently primer 1931 (C-T mis-match) was replaced with mutant primer 2072 (G-T mis-match) which detected only for the 621+1 mutation. Primer 2072 was included in the Standard B-Mix at 0.5μM.

(ii) G542X Mutant Primer (A-Mix)

Whilst carrier status for the G542X mutation could be detected using the Standard ARMS Test, the yield of mutant product was on occasion markedly reduced. In order to safe-guard against a false negative result an alternative G542X mutant primer containing no additional mis-match at the 3′ end was evaluated. Primer 2086 (no mis-match) consistantly produced a high yield of mutant product (whilst still retaining specificity for G542X mutant sequence) and conseqently was substituted into the Standard ARMS Test in place of primer 1949.

(iii) Elimination of Primer-dimer (A- and B-Mixes)

Primer-dimer bands (assumed to result from 3' homology between 2 or more component primers) were frequently observed throughout the development of the Standard ARMS multiplex. Whilst the presence of these primer-dimer bands did not necessarily affect the final diagnosis they did present a potential problem in that the available amount of particular ARMS primers would be variable from reaction to reaction. Conseqently, certain component ARMS reactions would not be optimal and the yield of ARMS product compromised. Each of the A- and B-Mix ARMS primers were analysed fro complementary at their 3' ends and strong homology between the 621+1-C and DF508-C primers was identified. A new 621+1-C primer, 2073, which was no longer complementary to DF508 common primer 1712 was substituted into the Standard A and B mixes. This single change prevented the formation of primer-dimers and resulted in overall increased yields of ARMS products.

The revised Standard ARMS Multiplex is summarised in Table 5 and an example of ARMS analyses using this test are shown in Figure 12.

To investigate the response of the modified standard test to changes in reaction conditions, the denaturation, annealing and extension temperatures were altered.

Denaturation Temperature (94°C)

Limits 84°C > (94°C), 96°C

At 84°C denaturation, diagnoses are correct and of reasonable intensity, however below this temperature 621+1 normal and mutant product bands are faint.

At 96°C denaturation, diagnoses are correct however all product bands are faint. Above this temperature no product bands are formed.

Annealing Temperature (60°C)

Limits 56°C > (60°C), 62°C

At 56°C annealing, diagnoses are correct and of reasonable intensity although primer dimers are beginning to form. Below this temperature non specific products are formed both larger thatn 621+1 and smaller than F508.

At 62°C annealing, diagnoses are correct however the intensity of G551D normal product band is slightly reduced. Above this temperature G551D normal, F508 normal and F508 mutant product bands are faint.

Extension Temperature (72°C)

Limits 62°C > (72°C), 76°C

At 62°C extension, diagnoses are correct and intensity is reasonable Lower temperatures have not been investigated in this study.

At 76°C extension, diagnoses are correct however overall intensity of products is low especially 621 + 1 normal and mutant products. Above this temperature results are too faint to interpret.

Effect of alterint all seqment temperatures simultaneously

Raising denaturation, annealing and extension temperature by 2°C (i.e to 96°C, 62°C, 74°C) results in low intensity of product, diagnoses are not possible.

Lowering denaturation, annealing and extension temperature by 2°C (i.e. to 92°C, 58°C, 70°C) has no effect on diagnoses or intensity of product bands.

Lowering denaturation, annealing and extension temperature by 4°C (i.e. to 90°C, 56°C, 68°C) results in the formation of non-specific large product bands.

It can be seen from these data that the test functions within wide temperature windows. The level of day to day variation of the thermal cycling devices used in these experiments falls within the acceptable temperature limits.

**Example 7**

**Further Development of the Standard ARMS Test to Include the Detection of R553X and W128X Mutations - The Standard+ Test.**

A) <u>R553X</u>

The development of an overARMS test for the simultaneous detection of the closely linked mutations G542X and G551D has already been described.

The incidence of the R553X mutation, also located in exon 11 and closely linked to the G551D and G542X mutations, is significant in CF affected individuals. As such, a method which would allow the simultaneous detection of all 3 mutations would prove valuable in determining CF carrier status.

The simultaneous detection of both G551D and R553X mutations presents two additional technical problems:

(i) direct competition of the G551D and R553X primers for target genomic DNA. (The G551D and R553X mutations are separated by only 5 nucleotides therefore the ARMS primers themselves overlap - a problem which was not encountered in the case of the G542X/G551D overARMS).

(ii) the G551D and R553X mutant PCR products would be difficult to distinguish by size difference using 3% agarose gels.

The latter problem was overcome by employing an elongated R553X mutant ARMS primer 60 bp in length (conventional ARMS primers normally 20-30 bp) thereby creating a 39 bp size difference between the expected G551D and R553X product bands. As such both ARMS products could be clearly distinguished using a 3% NuSieve gel.

Initially, a 60 bp mutant ARMS primer (2134) containing an additional G-G destabilising mismatch at the -2 position of the 3′ end but otherwise totally homologous to target DNA sequence, was included in the Standard ARMS 'A'-mix at 1μM. R553X mutant product was detected and the ARMS primer was specific for only mutant DNA seqence. The 621+1 normal, DF508 normal and G542X mutant ARMS products were unaffected by inclusion of the R553X primer but G551D mutant product was no longer visible. It appeared that the R553X mutant primer bound more effectively to target DNA than the G551D mutant primer thereby preventing any formation of G551D ARMS product. Any further destabilisation of the R553X mutant primer at the 3′ end (which would allow the G551D mutant primer to bind target DNA also) was likely to compromise the yield of R553X mutant product. Likewise, reducing the severity of the G551D mutant primer mis-match was likely to compromise specificity.

A second R553X mutant ARMS primer (2150) which was no longer completely homologous to target DNA at the distal (5′) end (9/30 bp random homology) but was otherwose identical to the original 2134 primer at the proximal (3′) end (30/30 bp homology) was also evaluated. When the 2150 R553X mutant ARMS primer (non-homologous 5′ tail) was included in the Standard 'A' reaction mix both R553X <u>and</u> G551D mutant products were detected i.e. the increased 5′ destabilisation of the R553X mutant primer enabled the G551D mutant primer to compete for target DNA. Again the 621+1, DF508 and G542X product bands were unaffected.

Although the R553X mutation could be easily detected using the method described above, the yield of R553X mutant product was generally lower than that observed for the other A-mix PCR products. In order to incease the amount of R553X product, several approaches were evaluated:-

(i) increasing R553X mutant primer concentration
(ii) reducing mis-match severity at -2 position
(iii) adding a secondary 'TAG' primer specific for the non homologous 5′ tail of the R553X mutant primer
(iv) incorporating a single mis-match at the -3 position of the R553X mutant primer

The first two approaches were unsuccessful. A 4-fold increase in the concentration of primer 2150 had only a marginal effect on product yield. Mutant primer 2227 (G-T mis-match at -2 position) was found to be more destabilising than primer 2150 (G-G mis-match) and resulted in a reduced yield of mutant product. Primer 2172 (no additional 3′ destabilising mis-match) gave increased amounts of product but was no longer specific for R553X mutant target sequence.

In a further attempt to increase the yield of R553X mutant product a TAG primer (2173) completely homologous for the 5′ 30 bp tail of primer 2150 was included in the A-Mix together with the 2150 primer itself. The combined use of the ARMS specific tailed primer and the corresponding TAG primer (both at 1μM concentration) resulted in a visible increase in the amount of R553X mutant product formed. Increasing the TAG : tailed ARMS primer ratio appeared to further increase the product yield. The optimal result was achieved when ARMS primer 2150 was included in the reaction mix at 1μM and the corresponding TAG primer 2173 at 3μM.

Whilst initial studies demonstrated that the TAG/tailed ARMS primer system was generally effective in increasing product yield, on occasion faint R553X mutant bands were still observed. In a final attempt to achieve a system whereby the product yield was consistently high, tailed ARMS primers containing either an A-A or C-A mis-match at the -3 position (2226 and 2225) respectively were evaluated. In the first instance, the TAG primer was not included in the reaction. Both primers generated increased yields of R553X mutant product and the band intensity was similar to that observed for the other A-mix ARMS products. There was little improvement when TAG 2173 was also included in the reaction mix and in the case of primer 2225 the addition of TAG primer comprised specificity.

The final concentration of the exon 11 common primer 1823 was unchanged ($2\mu M$).

G551D-N as a Control for R553X Homozygotes

A compound heteroxzygote individual for both G551D and R553X mutations was diagnosed as a G551D homozygote using the Standard ARMS test. (The G551D normal ARMS primer did not recognise R553X mutant sequence). This result could be predicted since the G551D normal primer already contained 2 mis-matches at the -2 and -3 positions of the target sequence and against R553X mutant sequence a third mis-match would be created at the -6 position.

In the Standard+ test this individual was correctly diagnosed as G551D/R553X, however the corresponding G551D normal ARMS product (B-Mix) was not observed. The disappearance of G551D normal product therefore acts as a control for R553X mutant sequence, in particular R553X homozygosity. An R553X homozygote sample was not available for analysis but we predict that this would evoke a similar situation to that observed with G551D/R553X individuals. This finding eliminates the need for a normal R553X control reaction in the Standard+ B-Mix.

B) W1282X

The development of a single ARMS test for the detection of the W1282X mutation has been described previously (Example 5).

The W1282X ARMS test was multiplexed into the Standard+ test such that the mutant reaction was included in the A-Mix (621+1-N, G551D-M, G542X-M, DF508-N, R553X-M) and the normal reaction in the B-Mix (621+1-M, G551D-N, G542X-N, DF508-M).

(i) Optimisation of the W1282X Mutant Reaction (A-Mix)

Mutant ARMS primers 2011 (C-A mis-match), 2012 (G-A) and 2109 (A-A) were initially evaluated at a concentration of $1\mu M$ in the Standard+ A-Mix. All 3 primers were used in conjunction with common primer 2174 to form a 202 bp product.

Primer 2011 (C-A) was not specific and mutant product was observed in normal individuals. The specificity was not improved by reducing the ARMS primer concentration to $0.5\mu M$. In contrast, primer 2012 (G-A) was too destabilising and mutant product was not visible in W1282X control samples.

Primer 2109 (A-A) was specific for W1282X mutant sequence and produced the desired yield of mutant product without adversely affecting the other standard+ A-Mix ARMS products.

(ii) Optimisation of the W1282X Normal Reaction (B-Mix)

In the first instance, normal and ARMS primers 2010 (C-A mis-match), 2013 (G-A) and 2155 (A-A) were evaluated in the Standard+ B-Mix at a concentration of $1\mu M$. All three primers generated copious amounts of normal product and comprised the G551D normal and G542X normal reactions to such an extent that the ARMS products, G551D in particular, could barely be detected. The problem was less pronounced with primer 2013 and by reducing the concentration of this primer together with common primer 2174 to 0.125 $\mu M$ it was possible to detect all other B-Mix ARMS products.

Diagnosis of G542X Homozygotes by Inclusion of the W1282X ARMS Reaction in the Standard+ Test

A faint non-specific band was routinely observed when G542X homozygous DNA was analysed using the Standard ARMS test. This observation was probably as a result of G542X normal primer priming G551D normal product (present in abundance). The introduction of a second competing ARMS reaction in the Standard+ B-Mix (W1282X normal ARMS reaction) had the desired effect of preventing or at least minimising this non-specific priming and hence G542X homozygote individuals could be correctly and unequivocably diagnosed.

C) R1283M

The R1283M mutation is a G to T substitution at position 3980 in exon 20 of the CF gene (Cheadle et al, 1991) and can be detected using W1282X mutant ARMS primer 2109 (A-A mis-match).

We believe that the detection of R1283M by primer 2109 is an example of where annealing at the 3' end of the ARMS primer occurs due to a loop-out elsewhere in its target/primer interaction. The inclusion of an A at -2 of the W1282X primer encourages the annealing of the 3' end with DNA carrying the R1283M sequence. That this annealing does indeed occur is demonstrated by the presence of a FokI site in the R1283M ARMS product. This restriction site would not be found if looping out of the primer had not occurred.

The primers used in the Standard+ multiplex are summarised in Table 6 and examples of ARMS analyses shown in Figure 13.

## Example 8

### Development of the extended ARMS test

The Extended mix initially consisted of four ARMS reactions multiplexed together, these were: N1303K, R560T, R117H and 1717-1G>A

As with the Standard test normal and mutant primers from different primer sets were combined in the same reaction.

The primers included in the A tube were; N1303K common 1874 and normal 1872, R560T common 1748 and mutant 1749, R117H common 1753 and normal 1838 and 1717-1G>A common 1748 and mutant 2069.

The primers included in the B tube were; N1303K common 1874 and mutant 1873, R560T common 1748 and normal 1747, R117H common 1753 and mutant 1832 and 1717-1G>A common 1748 and normal 2067. The product sizes of these ARMS reactions were; N1303K - 342bp, R560T - 316bp, R117H -237bp and 1717-1G>A - 220bp. Primers for R560T and 1717-1G>A are amplified using the same common primer, C1753, in an overARMS reaction.

### R117H

Primers C1753, N1838 and M1832 were included as part of the multiplex. When tested using DNA from an R117H heterozygote the mutant product was absent suggesting that the C-C mismatch (mm) was too destabilising. Primers N1834 and M1837 were substituted. The mutant product was still absent suggesting that the C-T mm was also too severe. Primers N1836 and M1835 with a C-A mm were then used. These primers worked well in the multiplex and gave correct diagnoses.

### 1717-1G>A and R560T

The primers initially used for 1717-1G>A were G1748, N2067 and M2069. The product yield and specificity were satisfactory although the product size was too large in comparison to the other multiplex products. The primers initially used for R560T were C1748, N1749 and M1747. The product was too faint suggesting that the A-G mm was too severe when used in an overARMS reaction with 1717-1G>A. Primers N2039 and M2040 with a weaker G-T mm were tried. The product band intensity was improved although still faint compared to the shorter overARMS product 1717-1G>A. The common primer for R560T and 1717-1G>A was changed to C222, this gave shorter products of 278bp and 180bp for R560T and 1717-1G>A respectively. Using the new 2222 band, intensities of R560T and 1717-1G>A were satisfactory.

### N1303K

Primers C1874, N1872 and M1873 were initially tried in the multiplex. The resultant 343bp product was faint. The primers were changed so that the ARMS primers now annealed to the opposite side of the N1303K mutation. The G+C content was higher than the previous primers and an additional C-T mm was included at the penultimate base. These primers were C1867, N1868 and M1869. No improvment in band intensity was seen.

### DI507/DF508

At this point DI507/DF508 primers C1876, N1878 and M1877 were added to the multiplex.

These primers gave satisfactory products although the N1303K product became fainter possibly due to the additional ARMS reaction in each tube. At this stage the extended mix consisted of the following primers.

The primers included in the A tube were:
N1303K common 1867 and normal 1868,
R560T common 2222 and mutant 2039,
R1117H common 1753 and normal 1836,
1717-1G>A common 2222 and mutant 2069
and DI507/DF508 common 1876 and normal 1878.

The primers included in the B tube were:
N1303K common 1867 and mutant 1869,
R560T common 2222 and normal 2040,
R117H common 1753 and mutant 1835,
1717-1G>A common 2222 and normal 2067
and DI507/DF508 common 1876 and mutant 1877.

The product sizes of these ARMS reactions were:
N1303K - 328bp, R560T - 278bp - 171701G>A - 180bp and DI507/DF508 - 146bp.

Enzyme and nucleotide concentrations were doubled to try to increase the amount of N1303K product but no improvement was seen.

New N1303K common primers C2240 and C2241 were synthesized to give smaller products of 118bp and 123bp respectively, in an attempt to improve band intensity. The resultant product band intensities showed a slight improvement. The concentration of N1303K primers was then doubled, band intensity did not alter significantly.

Tailed Amplification Primers (TAPs)

Primers with non-homologous GC rich tails were synthesized in an attempt to increase the stability of primer template interaction. These were C2259, N2257 and M2259. The effect of adding a GC rich TAG sequence (2212) homologous to the tail sequence was also teste.

The addition of GC rich tails resulted in enhanced N1303K product but addition of the TAG primer gave no additional imporvement. The resultant product from the tailed primers was however too large in comparison to other PCR products. A new tailed common primer C2283 was synthesized giving a 110bp product. To determine whether the enhanced yield of product using primer 2283 was due to the GC rich tail sequence, a second common primer of the same length and with the same 3' end but completely homologous to genomic DNA sequence was synthesised (2292). This primer also gave enhanced PCR product yield.

To increase further the size differential between the N1303K product and the F508 product a further tailed common primer (2298( which gave rise to a 90bp product and this primer was included in the final mix.

The resultant format for the extended mix is described below.
Primers in the A tube include:
R560T common 2222 and normal 2040,
R117H common 1753 and mutant 1835,
1717-1G>A common 2222 and normal 2067,
DI507/DF508 common 1876 and mutant 1877 and
N1303K common 2283 and normal 2257
Primers in the B tube include:
R560T common 2222 and mutant 2039,
R117H common 1753 and normal 1836,
1717-1G>A common 2222 and mutant 2069,
DI507/DF508 common 1876 and normal 1878 and
N1303 common 2298 and mutant 2258.

The product sizes are:
R560T - 278bp, R117H - 237bp, 1717-1G>A - 180bp, DI507/DF508 - 146bp and N1303K - 90bp.
All primers are present at a concentration of 1μM.
An example of this multiplex is shown in Figure 14.

# TABLE 1 ARMS Oligonucleotide Primers

| MUTATION | EXON | PRIMER | SPECIFICITY | MIS—MATCH |
|---|---|---|---|---|
| F508/I507 | 10 | 1712 | C | |
| | | 1711 | N | |
| | | 1710 | M | |
| | | 1880 | N | |
| | | 1879 | M | |
| F508 | 10 | 1876 | C | |
| | | 1878 | N | |
| | | 1877 | M | |
| R560T | 11 | 1748 | C | |
| | | 1747 | N | G—A |
| | | 1749 | M | G—A |
| R117H | 4 | 1753 | C | |
| | | 1836 | N | C—A |
| | | 1835 | M | C—A |
| | | 1834 | N | C—T |
| | | 1837 | M | C—T |
| | | 1838 | N | C—C |
| | | 1832 | M | C—C |
| G542X | 11 | 1823 | C | |
| | | 1830 | N | G—A |
| | | 1831 | M | G—A |
| | | 1949 | M | G—G |
| R553X | 11 | 1823 | C | |
| | | 1853 | N | G—A |
| | | 1854 | M | G—A |

# TABLE 1 (CONT'D)

| MUTATION | EXON | PRIMER | SPECIFICITY | MIS—MATCH |
|----------|------|--------|-------------|-----------|
| G551D | 11 | 1823 | C | |
| | | 1821 | N | C—T |
| | | 1822 | M | C—T |
| | | 1841 | N | G—T(−2),C—T(−3) |
| | | 1842 | N | C—T(−2),C—T(−3) |
| | | 1843 | N | C—T(−2),C—A(−3) |
| | | 1847 | N | C—T(−3) |
| N1303K | 21 | 1850 | C | |
| | | 1852 | N | C—T |
| | | 1851 | M | C—T |
| | | 1874 | C | |
| | | 1870 | N | G—T |
| | | 1871 | M | G—T |
| | | 1872 | N | |
| | | 1873 | M | |
| | | 1867 | C | |
| | | 1868 | N | C—T |
| | | 1869 | M | C—T |
| 621+1 | 4 | 1934 | C | |
| | | 1933 | N | C—T |
| | | 1931 | M | C—T |
| * | | 1713 | | |
| ** | | 1714 | | |

\* AAT CONTROL PRIMER 1
\*\* AAT CONTROL PRIMER 2

Table 1 __CF ARMS PRIMER SEQUENCES__

| MUTATION | PRIMER | SEQUENCE (5'-> 3') | CODE |
|---|---|---|---|
| F508 | 1712 | GACTTCACTTCTAATGATGATTATGGGAGA | DF-C |
| | 1711 | TATATTCATCATAGGAAACACCAAAGATGA | |
| | 1710 | TATATTCATCATAGGAAACACCAATGATAT | |
| | 1880 | GTATCTATATTCATCATAGGAAACACCACA | DF-j-N |
| | 1879 | GTATCTATATTCATCATAGGAAACACCATT | DF-w-M |
| F508/I507 | 1876 | GGGTAGTGTGAAGGGGTTCATATGCATAATC | DF/DI-C |
| | 1878 | GCCTGGCACCATTAAAGAAAATATCATCTT | DF/DI-N |
| | 1877 | GCCTGGCACCATTAAAGAAAATATCATTGG | DF/DI-b5-M |
| R560T | 1748 | AAAATTTCAGCAATGTTGTTTTTGACCAAC | RT-C |
| | 1747 | GCTTGCTAGACCAATAATTAGTTATTCAAC | RT-e-N |
| | 1749 | GCTTGCTAGACCAATAATTAGTTATTCAAG | RT-e-M |
| | 2222 | GCATTTGAAATAATGGAGATGCAATG | RT-C2 |
| | 2040 | GCTTGCTAGACCAATAATTAGTTATTCATC | RT-v-N |
| | 2039 | GCTTGCTAGACCAATAATTAGTTATTCATG | RT-v-M |
| R117H | 1753 | CACATATGGTATGACCCTCTATATAAACTC | RH-C |
| | 1836 | CCTATGCCTAGATAAATCGCGATAGAAC | RH-d-N |
| | 1835 | CCTATGCCTAGATAAATCGCGATAGAAT | RH-d-M |
| | 1834 | CCTATGCCTAGATAAATCGCGATAGATC | RH-s-N |
| | 1837 | CCTATGCCTAGATAAATCGCGATAGATT | RH-s-M |
| | 1838 | CCTATGCCTAGATAAATCGCGATAGACC | RH-h-N |
| | 1832 | CCTATGCCTAGATAAATCGCGATAGACT | RH-h-M |
| G542X | 1823 | TAAAATTTCAGCAATGTTGTTTTTGACC | GX-C |
| | 1830 | ACTCAGTGTGATTCCACCTTCTAC | GX-e-N |
| | 1831 | ACTCAGTGTGATTCCACCTTCTAA | GX-e-M |
| | 1948 | CACTCAGTGTGATTCCACCTTCTTA | GX-v-M |
| | 1949 | CACTCAGTGTGATTCCACCTTCTGA | GX-q-M |
| | 2086 | CACTCAGTGTGATTCCACCTTCTCA | GX-M |
| R553X | 1853 | CACCTTGCTAAAGAAATTCTTGCTAG | RX-e-N |
| | 1854 | CACCTTGCTAAAGAAATTCTTGCTAA | RX-e-M |
| | 2189 | TATTCACCTTGCTAAAGAAATTCTTGCTGA | RX-q-M |
| | 2134 | CAGCAAATGCTTGCTAGACCAATAATTAGT TATTCACCTTGCTAAAGAAATTCTTGCTGA | RX-q-M |
| | 2150 | gactgactgactgactgactctgactgact TATTCACCTTGCTAAAGAAATTCTTGCTGA | RX-q-M(T2) |
| | 2225 | gactgactgactgactgactctgactgact TATTCACCTTGCTAAAGAAATTCTTGCCCA | RX-f3-M(T2) |
| | 2226 | gactgactgactgactgactctgactgact TATTCACCTTGCTAAAGAAATTCTTGCACA | RX-b3-M(T2) |
| | 2180 | tcatgcgtccatggtccggaCAGCTAGCAG TATTCACCTTGCTAAAGAAATTCTTGCTGA | RX-q-M(T1) |
| | 2227 | gactgactgactgactgactctgactgact TATTCACCTTGCTAAAGAAATTCTTGCTTA | RX-v-M(T2) |
| | 2228 | gcgaccggtcgccggacgccctgactgact TATTCACCTTGCTAAAGAAATTCTTGCTGA | RX-q-M(T3) |
| | 2172 | gactgactgactgactgactctgactgact TATTCACCTTGCTAAAGAAATTCTTGCTCA | RX-M(T2) |
| | 2175 | tcatgcgtccatggtccggaCAGCTAGCAG TATTCACCTTGCTAAAGAAATTCTTGCTCA | RX-M(T1) |
| | 2176 | tcatgcgtccatggtccggaCAGCTAGCAG TATTCACCTTGCTAAAGAAATTCTTGCTCG | RX-N(T1) |

|  |  |  |  |
|---|---|---|---|
|  | 2177 | tcatgcgtccatggtccggaCAGCTAGCAG TATTCACCTTGCTAAAGAAATTCTTGCTGG | RX-L-N(T1) |
| G551D | 1821 | GCTAAAGAAATTCTTGCTCGTTGCC | GD-J-N |
|  | 1822 | AGCTAAAGAAATTCTTGCTCGTTGCT | GD-J-M |
|  | 1841 | GCTAAAGAAATTCTTGCTCGTTTGC | GD-r2s3-N |
|  | 1842 | GCTAAAGAAATTCTTGCTCGTTTCC | GD-j2s3-N |
|  | 1843 | GCTAAAGAAATTCTTGCTCGTTACC | GD-j2d3-N |
|  | 1847 | GCTAAAGAAATTCTTGCTCGTTTAC | GD-s3-N |
| N1303K | 1850 | GTTGGTATGAGTTACCCCTTTCAAAATC | NK-C |
|  | 1852 | TTTATTTTTTCTGGAACATTTAGAAAAACC | NK-j-N |
|  | 1851 | TTTATTTTTTCTGGAACATTTAGAAAAACG | NK-j-M |
|  | 1874 | CATGCACACAAAGTGTGTAGAATGATC | NK-C2 |
|  | 1870 | TTTATTTTTTCTGGAACATTTAGAAAAAGC | NK-r-N |
|  | 1871 | TTTATTTTTTCTGGAACATTTAGAAAAAGG | NK-r-M |
|  | 1872 | TTTATTTTTTCTGGAACATTTAGAAAAAAC | NK-N |
|  | 1873 | TTTATTTTTTCTGGAACATTTAGAAAAAAG | NK-M |
|  | 1867 | CTCAATTTCTTTATTCTAAAGACATTGG | NK~-C |
|  | 1868 | GATCACTCCACTGTTCATAGGGATCCACG | NK~-j-N |
|  | 1869 | GATCACTCCACTGTTCATAGGGATCCACC | NK~-j-M |
|  | 2240 | TAAAAAGTTATTTAAGTTATTCATACTTTCTTCTTC | NK~-C2 |
|  | 2241 | GAGTTACCCCTTTCAAAATCATTTCAGTTAGCAG | NK-C3 |
|  | 2259 | gcgaccggtcgccggacgccTCATACTTTCTTCTT CTTTTCTTTTTTGCTATAGAA | NK~-C3(T3) |
|  | 2283 | gcgaccggtcgccggacgccTCTTCTTCTTTTCTT TTTTGCTATAGAAAG | NK~-C4(T3) |
|  | 2257 | gcgaccggtcgccggacgccGATCACTCCACTGTT CATAGGGATCCACG | NK~-j-N(T3) |
|  | 2258 | gcgaccggtcgccggacgccGATCACTCCACTGTT CATAGGGATCCACC | NK~-j-M(T3) |
| 621+1G>T | 1934 | CACATATGGTATGACCCTCTATATAAACTC | 621-C |
|  | 1933 | TGCCATGGGGCCTGTGCAAGGAAGTATTCC | 621-j-N |
|  | 1931 | TGCCATGGGGCCTGTGCAAGGAAGTATTCA | 621-j-M |
|  | 2071 | TGCCATGGGGCCTGTGCAAGGAAGTATTTA | 621-w-M |
|  | 2072 | TGCCATGGGGCCTGTGCAAGGAAGTATTGA | 621-r-M |
|  | 2073 | TCACATATGGTATGACCCTCTATATAAACT | 621-C2 |
| 1717-1G>A | 2065 | GTCTTTCTCTGCAAACTTGGAGATGTTC | 1717-v-N |
|  | 2066 | GTCTTTCTCTGCAAACTTGGAGATGTAC | 1717-e-N |
|  | 2067 | GTCTTTCTCTGCAAACTTGGAGATGTGC | 1717-q-N |
|  | 2070 | GTCTTTCTCTGCAAACTTGGAGATGTTT | 1717-v-M |
|  | 2069 | GTCTTTCTCTGCAAACTTGGAGATGTGT | 1717-q-M |
|  | 2068 | GTCTTTCTCTGCAAACTTGGAGATGTAT | 1717-e-M |
|  | 2038 | AAAATTTCAGCAATGTTGTTTTTGACCAAC | RT-C |
| W1282X | 2010 | CCTGTGGTATCACTCCAAAGGCTTTCCCC | WX-f-N |
|  | 2011 | CCTGTGGTATCACTCCAAAGGCTTTCCCT | WX-f-M |
|  | 2012 | CCTGTGGTATCACTCCAAAGGCTTTCCGT | WX-k-M |
|  | 2013 | CCTGTGGTATCACTCCAAAGGCTTTCCGC | WX-k-N |
|  | 2155 | CCTGTGGTATCACTCCAAAGGCTTTCCAC | WX-b-N |
|  | 2109 | CCTGTGGTATCACTCCAAAGGCTTTCCAT | WX-b-M |
|  | 2014 | CCCATCACTTTTACCTTATAGGTGGGCCTC | WX-C1 |
|  | 2174 | GTACCTATATGTCACAGAAGTGATCCCATC | WX-C2 |
| G85E | 2248 | CGGCGATGTTTTTTCTGGAGATTTATGTTCTATTG | GE-s-N |
|  | 2247 | CGGCGATGTTTTTTCTGGAGATTTATGTTCTATTA | GE-s-M |
|  | 2251 | CGGCGATGTTTTTTCTGGAGATTTATGTTCTATAG | GE-d-N |
|  | 2250 | CGGCGATGTTTTTTCTGGAGATTTATGTTCTATAA | GE-d-M |

|  | 2246 | ATTCACCAGATTTCGTAGTCTTTTCATAATC | GE-C |
| AAT | 677 | CCCACCTTCCCCTCTCTCCAGGCAAATGGG | AAT1 |
|  | 678 | GGGCCTCAGTCCCAACATGGCTAAGAGGTG | AAT2 |
|  | 1713 | TGTCCACGTGAGCCTTGCTCGAGGCCTGGG | AAT3 |
|  | 1714 | GAGACTTGGTATTTTGTTCAATCATTAAG | AAT4 |
| TAG | 2164 | tcatgcgtccatggtccgga | T1 |
|  | 2173 | gactgactgactgactgactctgactgact | T2 |
|  | 2212 | gcgaccggtcgccggacgcc | T3 |

## Mis-match Codes

|  | TEMPLATE SEQUENCE | | | |
|  |  | A | C | G | T -- |
| PRIMER SEQUENCE | A | B | D | E | - |
|  | C | F | H | - | J |
|  | G | K | - | Q | R |
|  | T | - | S | V | W |

### note on primer nomenclature

A large number of oligonucleotide PCR primers (amplimers) were used in this study. Each primer has been given a three part code of the form XX-y-Z. The first part refers to the CF mutation under study, for example R117H is abbreviated to RH and G551D to GD. The second part (y in the example above) indicates which additional mismatches (if any) have been included at the penultimate base of the amplimer. Each mismatch has a specific code shown above. If any additional mismatch is included at a position other than the penultimate base this is indicated by the inclusion of the base number after the mismatch code. The third part of the code indicates whether the primer is specific for the mutant sequence (M), normal sequence (N) or common to both (C). For example, RH-d-N indicates that this primer is used to analyse the R117H mutation (RH), that it has an A residue as the penultimate base which mis-matches a C residue in the target sequence (code d) and that it is specific for the normal allele (N).

A number of the primers contained non-homologous 5' tail sequences. These are indicated by the use of lower case type in the primer sequence and by the inclusion of a T-code in brackets at the end of the primer code. The tail sequences themselves, termed TAGS, are also shown along with their T-codes. The presence of a ~ in the code is used to indicate that these primers are complementary to the opposite strand of DNA compared to the other primers for the same mutation.

# TABLE 2 overARMS ANALYSES

| GENOTYPE | R560T—N | R553X—N | G542X—N | R560T—M | R553X—M | G542X—M |
|----------|---------|---------|---------|---------|---------|---------|
| R553X/? | + | — | + | — | + | — |
| R553X/G551D | + | — | + | — | + | — |
| R560T/F508 | + | — | + | + | — | — |
| R560T/G551D | + | — | + | + | — | — |
| G542X/F508 | + | + | + | — | — | + |
| G542X/? | + | + | + | — | — | + |
| R553X/F508 | + | — | + | — | + | — |
| +/+ (normal) | + | — | + | — | — | — |

\+ PRODUCT FORMED
\- PRODUCT NOT FORMED

# TABLE 3 STANDARD overARMS MULTIPLEX

|  | A — MIX | | | B — MIX | | |
| MUTATION | NORMAL | MUTANT | COMMON | NORMAL | MUTANT | COMMON |
|---|---|---|---|---|---|---|
| F508/I507 | 1880 | | 1712 | | 1879 | 1712 |
| G542X | | 1949 | 1823 | 1830 | | 1823 |
| G551D | | 1822 | 1823 | 1843 | | 1823 |
| 621+1 | 1933 | | 1934 | | 1931 | 1934 |

All primers used at 1.0 M except for:
1931, 1933, 1934 — 0.5 M
1843, 1879 — 2.0 M

## Table 4

### Cystic Fibrosis (CF) – Mutations

| Nucleotide Change | Amino Acid Change | Exon |
|---|---|---|
| G to C at 129 | none: regulation? | 1 |
| C to T at 247 | Gln to Stop at 39 | 2 |
| G to A at 386 | Gly to Glu at 85 | 3 |
| G to A at 403 | Gly to Arg at 91 | 3 |
| deletion of A at 444 | frameshift | 4 |
| G to C at 460 | Asp to His at 110 | 4 |
| G to A at 482 | Arg to His at 117 | 4 |
| T to A at 498 | Tyr to Stop at 122 | 4 |
| deletion of A at 556 | frameshift | 4 |
| deletion of A at 574 | frameshift | 4 |
| T to C at 575 | Ile to Thr at 148 | 4 |
| G to T at 621+1 | splice mutation | intron 1 |
| G to A at 664 | Gly to Arg at 178 | 5 |
| G to T at 711+1 | splice mutation | intron 5 |
| T to G at 729 | His to Gln at 199 | 6a |
| deletion of 22 bp | frameshift | 6a |
| G to A at 1022 | Arg to Gln at 297 | 7 |
| C to T at 1132 | Arg to Trp at 334 | 7 |
| TC insertion after 1154 | frameshift | 7 |
| G to C at 1172 | Arg to Pro at 347 | 7 |
| deletion of T at 1213 | frameshift | 7 |
| GC at 1342-1 | splice mutation | intron 8 |
| C to A at 1496 | Ala to Glu at 455 | 9 |
| G to T at 1505 | Gly to Val at 458 | 9 |
| C to T at 1609 | Gln to Stop at 493 | 10 |
| 3bp deletion | deletion of Ile at 506/7 | 10 |
| 3bp deletion | deletion of Phe at 508 | 10 |
| deletion of TA at 1677 | frameshift | 10 |
| G to T at 1690 | Val to Phe at 520 | 10 |

Table 4 (cont'd)                                              2 of 3

Cystic Fibrosis (CP) - Mutations

| Nucleotide Change | Amino Acid Change | Exon |
|---|---|---|
| C to A at 1705 | Cys to Stop at 524 | 10 |
| G to A at 1717-1 | splice mutation | intron 10 |
| G to T at 1756 | Gly to Stop at 542 | 11 |
| A to C at 1777 | Ser to Arg at 549 | 11 |
| G to A at 1778 | Ser to Asn at 549 | 11 |
| G to T at 1778 | Ser to Ile at 349 | 11 |
| T to G at 1779 | Ser to Arg at 549 | 11 |
| G to A at 1784 | Gly to Asp at 551 | 11 |
| deletion of G at 1784 | frameshift | 11 |
| C to T at 1786 | Gln to Stop at 552 | 11 |
| C to T at 1789 | Arg to Stop at 553 | 11 |
| G to A at 1790 | Arg to Gln at 553 | 11 |
| G to A at 1807 | Ala to Thr at 559 | 11 |
| G to C at 1811 | Arg to Thr at 560 | 11 |
| T to A at 1819 | Tyr to Asn at 563 | 12 |
| C to A at 1853 | Pro to His at 574 | 12 |
| C insertion after 2522 | frameshift | 13 |
| AT insertion after 2556 | frameshift | 13 |
| G to A at 2670 | Trp to Stop at 846 | 14a |
| C to T at 2683 | Arg to Stop at 851 | 14a |
| G to A at 2789 | splicing mutation | intron 14b |
| A to G at 2870 | Tyr to Cys at 913 | 15 |
| deletion of T at 2909 | frameshift | 15 |
| T to C at 3362 | Leu to Pro at 1077 | 17b |
| C to A at 3408 | Tyr to Stop at 1092 | 17b |
| A to G at 3600-2 | splice mutation | intron 18 |
| C to T at 3616 | Arg to Stop at 1162 | 19 |
| deletion of C at 3659 | frameshift | 19 |
| deletion of A at 3662 | frameshift | 19 |

Table 4 (cont')

## Cystic Fibrosis (CF) – Mutations

| Nucleotide Change | Amino Acid Change | Exon |
| --- | --- | --- |
| deletion of A at 3732 | frameshift | 19 |
| A to G at 3730 | | 19 |
| G to A at 3739 | Ile to Val at 1203 | 19 |
| deletion of T at 3821 | frameshift | 19 |
| T to G at 3850 | splice mutation | intron 19 |
| C to A at 3863 | Gly to Glu at 1244 | 20 |
| C to A at 3896 | Ser to Stop at 1255 | 20 |
| G to A at 3978 | Trp to Stop at 1282 | 20 |
| G to C at 1005 | Gln to His at 1291 | 20 |
| C to G at 4041 | Asn to Lys at 1303 | 21 |
| G to A at 4079 | Trp to Stop at 1316 | 21 |
| G to A at 4178 | Gly to Asp at 1349 | 22 |
| G to T at 4243 | Glu to Stop at 1371 | 22 |
| G to A at 4374+1 | splice mutation | 23 |

TABLE 5     <u>REVISED STANDARD overARMS MULTIPLEX</u>

| MUTATION | A - MIX | | | B - MIX | | |
|---|---|---|---|---|---|---|
| | NORMAL | MUTANT | COMMON | NORMAL | MUTANT | COMMON |
| F508 | 1880 | | 1712 | | 1879 | 1712 |
| G542X | | 2086 | 1823 | 1830 | | 1823 |
| G551D | | 1822 | 1823 | 1843 | | 1823 |
| 621+1 | 1933 | | 2073 | | 2072 | 2703 |

All primers used at 1.0μM except for:

1931, 1933, 1934 - 0.5μM
1843, 1879 - 2.0μMM

TABLE 6          STANDARD + MULTIPLEX

| | A – MIX | | | B – MIX | | |
|---|---|---|---|---|---|---|
| MUTATION | NORMAL | MUTANT | COMMON | NORMAL | MUTANT | COMMON |
| 621+1 | 1933 | | 2073 | | 2072 | 2073 |
| R553X | | 2225 | 1823 | | | |
| G551D | | 1822 | 1823 | 1843 | | 1823 |
| G542X | | 2086 | 1823 | 1830 | | 1823 |
| W1282X | | 2109 | 2174 | 2013 | | 2174 |
| DF508 | 1880 | | 1712 | | 1879 | 1712 |

All primers used at 1µM except for:

1933, 2073, 2072 – 0.5µM
2013, 2174 – 0.125µM  (B-Mix only)
1843, 1879 – 2µM
1823 – 2µM final concentration  (A- and B-Mixes)

## SEQUENCE LISTING

(1) GENERAL INFORMATION

    (i)        APPLICANT: Imperial Chemical Industries PLC

    (ii)      TITLE OF INVENTION: METHOD OF DETECTION

    (iii)     NUMBER OF SEQUENCES: 97

    (iv)     CORRESPONDENCE ADDRESS:

            (A)   ADDRESSEE: Legal Department: Patents

            (B)   STREET: Bessemer Road

            (C)   CITY: Welwyn Garden City

            (D)   STATE: Hertfordshire

            (E)   COUNTRY: United Kingdom

            (F)   ZIP: GB-AL7 1HD

    (v)       COMPUTER READABLE FORM:

            (A)   MEDIUM TYPE: Diskette, 3.50 inch, 1.2 Mb

                              storage

            (B)   COMPUTER: 18 M PS/2

            (C)   OPERATING SYSTEM: PC-DOS 3.20

            (D)   SOFTWARE: ASCII from WPS-PLUS

    (vi)      CURRENT APPLICATION DATA:

            (A)   APPLICATION NUMBER

            (B)   FILING DATE:

            (C)   CLASSIFICATION:

    (vii)     PRIOR APPLICATION DATA:

            (A)   APPLICATION NO. 9102148.5

            (B)   FILING DATE: 31-Jan-1991

            (A)   APPLICATION NO. 9126085.1

            (B)   FILING DATE: 06-Dec-1991

(2)     INFORMATION FOR SEQ ID NO:1:

        (i)   SEQUENCE CHARACTERISTICS:
             (A) LENGTH: 30
             (B) TYPE: Nucleic Acid
             (C) STRANDEDNESS: Single
             (D) TOPOLOGY: Linear


GACTTCACTT CTAATGATGA TTATGGGAGA              30


(2)     INFORMATION FOR SEQ ID NO:2:

        (i)   SEQUENCE CHARACTERISTICS:
             (A) LENGTH: 30
             (B) TYPE: Nucleic Acid
             (C) STRANDEDNESS: Single
             (D) TOPOLOGY: Linear


TATATTCATC ATAGGAAACA CCAAAGATGA              30


(2)     INFORMATION FOR SEQ ID NO:3:

        (i)   SEQUENCE CHARACTERISTICS:
             (A) LENGTH: 30
             (B) TYPE: Nucleic Acid
             (C) STRANDEDNESS: Single
             (D) TOPOLOGY: Linear


TATATTCATC ATAGGAAACA CCAATGATAT              30

(2)    INFORMATION FOR SEQ ID NO:4:

    (i)   SEQUENCE CHARACTERISTICS:
         (A) LENGTH: 30
         (B) TYPE: Nucleic Acid
         (C) STRANDEDNESS: Single
         (D) TOPOLOGY: Linear

GTATCTATAT TCATCATAGG AAACACCACA                30

(2)    INFORMATION FOR SEQ ID NO:5:

    (i)   SEQUENCE CHARACTERISTICS:
         (A) LENGTH: 30
         (B) TYPE: Nucleic Acid
         (C) STRANDEDNESS: Single
         (D) TOPOLOGY: Linear

GTATCTATAT TCATCATAGG AAACACCATT                30

(2)    INFORMATION FOR SEQ ID NO:6:

    (i)   SEQUENCE CHARACTERISTICS:
         (A) LENGTH: 30
         (B) TYPE: Nucleic Acid
         (C) STRANDEDNESS: Single
         (D) TOPOLOGY: Linear

GGGTAGTGTG AAGGGTTCAT ATGCATAATC                30

(2)    INFORMATION FOR SEQ ID NO:7:

        (i)   SEQUENCE CHARACTERISTICS:
                (A) LENGTH: 30
                (B) TYPE: Nucleic Acid
                (C) STRANDEDNESS: Single
                (D) TOPOLOGY: Linear


GCCTGGCACC ATTAAAGAAA ATATCATCTT                                    30


(2)    INFORMATION FOR SEQ ID NO:8:

        (i)   SEQUENCE CHARACTERISTICS:
                (A) LENGTH: 30
                (B) TYPE: Nucleic Acid
                (C) STRANDEDNESS: Single
                (D) TOPOLOGY: Linear


GCCTGGCACC ATTAAAGAAA ATATCATTGG                                    30


(2)    INFORMATION FOR SEQ ID NO:9:

        (i)   SEQUENCE CHARACTERISTICS:
                (A) LENGTH: 30
                (B) TYPE: Nucleic Acid
                (C) STRANDEDNESS: Single
                (D) TOPOLOGY: Linear


AAAATTTGAG CAATGTTGTT TTTGACCAAC                                    30

(2)    INFORMATION FOR SEQ ID NO:10:

(i)    SEQUENCE CHARACTERISTICS:
                (A) LENGTH: 30
                (B) TYPE: Nucleic Acid
                (C) STRANDEDNESS: Single
                (D) TOPOLOGY: Linear


GCTTGCTAGA CCAATAATTA GTTATTCAAC                    30


(2)    INFORMATION FOR SEQ ID NO:11:


(i)    SEQUENCE CHARACTERISTICS:
                (A) LENGTH: 30
                (B) TYPE: Nucleic Acid
                (C) STRANDEDNESS: Single
                (D) TOPOLOGY: Linear


GCTTGCTAGA CCAATAATTA GTTATTCAAG                    30


(2)    INFORMATION FOR SEQ ID NO:12:


(i)    SEQUENCE CHARACTERISTICS:
                (A) LENGTH: 26
                (B) TYPE: Nucleic Acid
                (C) STRANDEDNESS: Single
                (D) TOPOLOGY: Linear


GCATTTGAAA TAATGGAGAT GCAATG                    26

EP 0 497 527 A1

(2)    INFORMATION FOR SEQ ID NO:13:

(i)   SEQUENCE CHARACTERISTICS:
            (A) LENGTH: 30
            (B) TYPE: Nucleic Acid
            (C) STRANDEDNESS: Single
            (D) TOPOLOGY: Linear


GCTTGCTAGA CCAATAATTA GTTATTCATC                     30


(2)    INFORMATION FOR SEQ ID NO:14

(i)   SEQUENCE CHARACTERISTICS:
            (A) LENGTH: 30
            (B) TYPE: Nucleic Acid
            (C) STRANDEDNESS: Single
            (D) TOPOLOGY: Linear


GCTTGCTAGA CCAATAATTA GTTATTCATG                     30


(2)    INFORMATION FOR SEQ ID NO:15:

(i)   SEQUENCE CHARACTERISTICS:
            (A) LENGTH: 30
            (B) TYPE: Nucleic Acid
            (C) STRANDEDNESS: Single
            (D) TOPOLOGY: Linear


CACATATGGT ATGACCCTCT ATATAAACTC                     30

(2)    INFORMATION FOR SEQ ID NO:16:

     (i)    SEQUENCE CHARACTERISTICS:

         (A) LENGTH: 28

         (B) TYPE: Nucleic Acid

         (C)· STRANDEDNESS: Single

         (D) TOPOLOGY: Linear

CCTATGCCTA GATAAATCGC GATAGAAC    28

(2)    INFORMATION FOR SEQ ID NO:17:

     (i)    SEQUENCE CHARACTERISTICS:

         (A) LENGTH: 28

         (B) TYPE: Nucleic Acid

         (C) STRANDEDNESS: Single

         (D) TOPOLOGY: Linear

CCTATGCCTA GATAAATCGC GATAGAAT    28

(2)    INFORMATION FOR SEQ ID NO:18:

     (i)    SEQUENCE CHARACTERISTICS:

         (A) LENGTH: 28

         (B) TYPE: Nucleic Acid

         (C) STRANDEDNESS: Single

         (D) TOPOLOGY: Linear

CCTATGCCTA GATAAATCGC GATAGATC    28

(2)     INFORMATION FOR SEQ ID NO:19:

        (i)   SEQUENCE CHARACTERISTICS:
                    (A) LENGTH: 28
                    (B) TYPE: Nucleic Acid
                    (C) STRANDEDNESS: Single
                    (D) TOPOLOGY: Linear


CCTATGCCTA GATAAATCGC GATAGATT                                    28


(2)     INFORMATION FOR SEQ ID NO:20:

        (i)   SEQUENCE CHARACTERISTICS:
                    (A) LENGTH: 28
                    (B) TYPE: Nucleic Acid
                    (C) STRANDEDNESS: Single
                    (D) TOPOLOGY: Linear


CCTATGCCTA GATAAATCGC GATAGACC                                    28


(2)     INFORMATION FOR SEQ ID NO:21:

        (i)   SEQUENCE CHARACTERISTICS:
                    (A) LENGTH: 28
                    (B) TYPE: Nucleic Acid
                    (C) STRANDEDNESS: Single
                    (D) TOPOLOGY: Linear


CCTATGCCTA GATAAATCGC GATAGACT                                    28

(2)    INFORMATION FOR SEQ ID NO:22:

    (i)   SEQUENCE CHARACTERISTICS:
             (A) LENGTH: 28
             (B) TYPE: Nucleic Acid
             (C) STRANDEDNESS: Single
             (D) TOPOLOGY: Linear

TAAAATTTCA GCAATGTTGT TTTTGACC                    28

(2)    INFORMATION FOR SEQ ID NO:23:

    (i)   SEQUENCE CHARACTERISTICS:
             (A) LENGTH: 24
             (B) TYPE: Nucleic Acid
             (C) STRANDEDNESS: Single
             (D) TOPOLOGY: Linear

ACTCAGTGTG ATTCCACCTT CTAC                        24

(2)    INFORMATION FOR SEQ ID NO:24:

    (i)   SEQUENCE CHARACTERISTICS:
             (A) LENGTH: 24
             (B) TYPE: Nucleic Acid
             (C) STRANDEDNESS: Single
             (D) TOPOLOGY: Linear

ACTCAGTGTG ATTCCACCTT CTAA                        24

(2)    INFORMATION FOR SEQ ID NO:25:

        (i)  SEQUENCE CHARACTERISTICS:
                (A) LENGTH: 25
                (B) TYPE: Nucleic Acid
                (C) STRANDEDNESS: Single
                (D) TOPOLOGY: Linear

CACTCAGTGT GATTCCACCT TCTTA                                    25

(2)    INFORMATION FOR SEQ ID NO:26:

        (i)  SEQUENCE CHARACTERISTICS:
                (A) LENGTH: 25
                (B) TYPE: Nucleic Acid
                (C) STRANDEDNESS: Single
                (D) TOPOLOGY: Linear

CACTCAGTGT GATTCCACCT TCTGA                                    25

(2)    INFORMATION FOR SEQ ID NO:27:

        (i)  SEQUENCE CHARACTERISTICS:
                (A) LENGTH: 25
                (B) TYPE: Nucleic Acid
                (C) STRANDEDNESS: Single
                (D) TOPOLOGY: Linear

CACTCAGTGT GATTCCACCT TCTCA                                    25

(2) INFORMATION FOR SEQ ID NO:28:

(i) SEQUENCE CHARACTERISTICS:
          (A) LENGTH: 26
          (B) TYPE: Nucleic Acid
          (C) STRANDEDNESS: Single
          (D) TOPOLOGY: Linear


CACCTTGCTA AAGAAATTCT TGCTAG                                    26


(2) INFORMATION FOR SEQ ID NO:29:

(i) SEQUENCE CHARACTERISTICS:
          (A) LENGTH: 26
          (B) TYPE: Nucleic Acid
          (C) STRANDEDNESS: Single
          (D) TOPOLOGY: Linear


CACCTTGCTA AAGAAATTCT TGCTAA                                    26


(2) INFORMATION FOR SEQ ID NO:30:

(i) SEQUENCE CHARACTERISTICS:
          (A) LENGTH: 30
          (B) TYPE: Nucleic Acid
          (C) STRANDEDNESS: Single
          (D) TOPOLOGY: Linear


TATTCACCTT GCTAAAGAAA TTCTTGCTGA CAGCAAATGC TTGCTAGACC AATAATTAGT   60

(2)     INFORMATION FOR SEQ ID NO:31:

        (i)   SEQUENCE CHARACTERISTICS:
                    (A) LENGTH: 60
                    (B) TYPE: Nucleic Acid
                    (C) STRANDEDNESS: Single
                    (D) TOPOLOGY: Linear


CAGCAAATGC TTGCTAGACC AATAATTAGT TATTCACCTT GCTAAAGAAA TTCTTGCTGA     60


(2)     INFORMATION FOR SEQ ID NO:32:

        (i)   SEQUENCE CHARACTERISTICS:
                    (A) LENGTH: 60
                    (B) TYPE: Nucleic Acid
                    (C) STRANDEDNESS: Single
                    (D) TOPOLOGY: Linear


GACTGACTGA CTGACTGACT CTGACTGACT TATTCACCTT GCTAAAGAAA TTCTTGCTGA     60


(2)     INFORMATION FOR SEQ ID NO:33:

        (i)   SEQUENCE CHARACTERISTICS:
                    (A) LENGTH: 60
                    (B) TYPE: Nucleic Acid
                    (C) STRANDEDNESS: Single
                    (D) TOPOLOGY: Linear


GACTGACTGA CTGACTGACT CTGACTGACT TATTCACCTT GCTAAAGAAA TTCTTGCCCA     60

(2)    INFORMATION FOR SEQ ID NO:34:

        (i)    SEQUENCE CHARACTERISTICS:
                    (A) LENGTH: 60
                    (B) TYPE: Nucleic Acid
                    (C) STRANDEDNESS: Single
                    (D) TOPOLOGY: Linear


GACTGACTGA CTGACTGACT CTGACTGACT TATTCACCTT GCTAAAGAAA TTCTTGCACA    60


(2)    INFORMATION FOR SEQ ID NO:35:

        (i)    SEQUENCE CHARACTERISTICS:
                    (A) LENGTH: 60
                    (B) TYPE: Nucleic Acid
                    (C) STRANDEDNESS: Single
                    (D) TOPOLOGY: Linear


TCATGCGTCC ATGGTCCGGA CAGCTAGCAG TATTCACCTT GCTAAAGAAA TTCTTGCTGA    60


(2)    INFORMATION FOR SEQ ID NO:36:

        (i)    SEQUENCE CHARACTERISTICS:
                    (A) LENGTH: 60
                    (B) TYPE: Nucleic Acid
                    (C) STRANDEDNESS: Single
                    (D) TOPOLOGY: Linear


GACTGACTGA CTGACTGACT CTGACTGACT TATTCACCTT GCTAAAGAAA TTCTTGCTTA    60

(2)     INFORMATION FOR SEQ ID NO:37:

(i)   SEQUENCE CHARACTERISTICS:
            (A) LENGTH: 60
            (B) TYPE: Nucleic Acid
            (C) STRANDEDNESS: Single
            (D) TOPOLOGY: Linear


GCGACCGGTC GCCGGACGCC CTGACTGACT TATTCACCTT GCTAAAGAAA TTCTTGCTGA     60


(2)     INFORMATION FOR SEQ ID NO:38:

(i)   SEQUENCE CHARACTERISTICS:
            (A) LENGTH: 60
            (B) TYPE: Nucleic Acid
            (C) STRANDEDNESS: Single
            (D) TOPOLOGY: Linear


GACTGACTGA CTGACTGACT CTGACTGACT TATTCACCTT GCTAAAGAAA TTCTTGCTCA     60


(2)     INFORMATION FOR SEQ ID NO:39:

(i)   SEQUENCE CHARACTERISTICS:
            (A) LENGTH: 60
            (B) TYPE: Nucleic Acid
            (C) STRANDEDNESS: Single
            (D) TOPOLOGY: Linear


TCATGCGTCC ATGGTCCGGA CAGCTAGCAG TATTCACCTT GCTAAAGAAA TTCTTGCTCA     60

(2)    INFORMATION FOR SEQ ID NO:40:

    (i)    SEQUENCE CHARACTERISTICS:
               (A) LENGTH: 60
               (B) TYPE: Nucleic Acid
               (C) STRANDEDNESS: Single
               (D) TOPOLOGY: Linear

TCATGCGTCC ATGGTCCGGA CAGCTAGCAG TATTCACCTT GCTAAAGAAA TTCTTGCTCG    60

(2)    INFORMATION FOR SEQ ID NO:41:

    (i)    SEQUENCE CHARACTERISTICS:
               (A) LENGTH: 60
               (B) TYPE: Nucleic Acid
               (C) STRANDEDNESS: Single
               (D) TOPOLOGY: Linear

TCATGCGTCC ATGGTCCGGA CAGCTAGCAG TATTCACCTT GCTAAAGAAA TTCTTGCTGG    60

(2)    INFORMATION FOR SEQ ID NO:42:

    (i)    SEQUENCE CHARACTERISTICS:
               (A) LENGTH: 25
               (B) TYPE: Nucleic Acid
               (C) STRANDEDNESS: Single
               (D) TOPOLOGY: Linear

GCTAAAGAAA TTCTTGCTCG TTGCC                                         25

(2)    INFORMATION FOR SEQ ID NO:43:

        (i)  SEQUENCE CHARACTERISTICS:
                    (A) LENGTH: 26
                    (B) TYPE: Nucleic Acid
                    (C) STRANDEDNESS: Single
                    (D) TOPOLOGY: Linear

AGCTAAAGAA ATTCTTGCTC GTTGCT                                      26

(2)    INFORMATION FOR SEQ ID NO:44:

        (i)  SEQUENCE CHARACTERISTICS:
                    (A) LENGTH: 25
                    (B) TYPE: Nucleic Acid
                    (C) STRANDEDNESS: Single
                    (D) TOPOLOGY: Linear

GCTAAAGAAA TTCTTGCTCG TTTGC                                       25

(2)    INFORMATION FOR SEQ ID NO:45:

        (i)  SEQUENCE CHARACTERISTICS:
                    (A) LENGTH: 25
                    (B) TYPE: Nucleic Acid
                    (C) STRANDEDNESS: Single
                    (D) TOPOLOGY: Linear

GCTAAAGAAA TTCTTGCTCG TTTCC                                       25

(2)    INFORMATION FOR SEQ ID NO:46:

    (i)   SEQUENCE CHARACTERISTICS:
          (A) LENGTH: 25
          (B) TYPE: Nucleic Acid
          (C) STRANDEDNESS: Single
          (D) TOPOLOGY: Linear

GCTAAAGAAA TTCTTGCTCG TTACC                                    25

(2)    INFORMATION FOR SEQ ID NO:47:

    (i)   SEQUENCE CHARACTERISTICS:
          (A) LENGTH: 25
          (B) TYPE: Nucleic Acid
          (C) STRANDEDNESS: Single
          (D) TOPOLOGY: Linear

GCTAAAGAAA TTCTTGCTCG TTTAC                                    25

(2)    INFORMATION FOR SEQ ID NO:48:

    (i)   SEQUENCE CHARACTERISTICS:
          (A) LENGTH: 28
          (B) TYPE: Nucleic Acid
          (C) STRANDEDNESS: Single
          (D) TOPOLOGY: Linear

GTTGGTATGA GTTACCCCTT TCAAAATC                                 28

(2)    INFORMATION FOR SEQ ID NO:49:

(i)    SEQUENCE CHARACTERISTICS:
            (A) LENGTH: 30
            (B) TYPE: Nucleic Acid
            (C) STRANDEDNESS: Single
            (D) TOPOLOGY: Linear

TTTATTTTTT CTGGAACATT TAGAAAAACC                    30

(2)    INFORMATION FOR SEQ ID NO:50:

(i)    SEQUENCE CHARACTERISTICS:
            (A) LENGTH: 30
            (B) TYPE: Nucleic Acid
            (C) STRANDEDNESS: Single
            (D) TOPOLOGY: Linear

TTTATTTTTT CTGGAACATT TAGAAAAACG                    30

(2)    INFORMATION FOR SEQ ID NO:51:

(i)    SEQUENCE CHARACTERISTICS:
            (A) LENGTH: 27
            (B) TYPE: Nucleic Acid
            (C) STRANDEDNESS: Single
            (D) TOPOLOGY: Linear

CATGCACACA AAGTGTGTAG AATGATC                    27

(2)    INFORMATION FOR SEQ ID NO:52:

        (i)  SEQUENCE CHARACTERISTICS:
                (A) LENGTH: 30
                (B) TYPE: Nucleic Acid
                (C) STRANDEDNESS: Single
                (D) TOPOLOGY: Linear

TTTATTTTTT CTGGAACATT TAGAAAAAGC          30


(2)    INFORMATION FOR SEQ ID NO:53:

        (i)  SEQUENCE CHARACTERISTICS:
                (A) LENGTH: 30
                  (B) TYPE: Nucleic Acid
                (C) STRANDEDNESS: Single
                (D) TOPOLOGY: Linear

TTTATTTTTT CTGGAACATT TAGAAAAAGG          30


(2)    INFORMATION FOR SEQ ID NO:54:

        (i)  SEQUENCE CHARACTERISTICS:
                (A) LENGTH: 30
                  (B) TYPE: Nucleic Acid
                (C) STRANDEDNESS: Single
                (D) TOPOLOGY: Linear

TTTATTTTTT CTGGAACATT TAGAAAAAAC          30

(2)    INFORMATION FOR SEQ ID NO:55:

(i)   SEQUENCE CHARACTERISTICS:
             (A) LENGTH: 30
             (B) TYPE: Nucleic Acid
             (C) STRANDEDNESS: Single
             (D) TOPOLOGY: Linear


TTTATTTTTT CTGGAACATT TAGAAAAAAG                    30


(2)    INFORMATION FOR SEQ ID NO:56:

(i)   SEQUENCE CHARACTERISTICS:
             (A) LENGTH: 28
             (B) TYPE: Nucleic Acid
             (C) STRANDEDNESS: Single
             (D) TOPOLOGY: Linear


CTCAATTTCT TTATTCTAAA GACATTGG                      28


(2)    INFORMATION FOR SEQ ID NO:57:

(i)   SEQUENCE CHARACTERISTICS:
             (A) LENGTH: 29
             (B) TYPE: Nucleic Acid
             (C) STRANDEDNESS: Single
             (D) TOPOLOGY: Linear


GATCACTCCA CTGTTCATAG GGATCCACG                     29

(2)    INFORMATION FOR SEQ ID NO:58:

        (i)    SEQUENCE CHARACTERISTICS:
                    (A) LENGTH: 29
                    (B) TYPE: Nucleic Acid
                    (C) STRANDEDNESS: Single
                    (D) TOPOLOGY: Linear


GATCACTCCA CTGTTCATAG GGATCCACC                                    29


(2)    INFORMATION FOR SEQ ID NO:59:

        (i)    SEQUENCE CHARACTERISTICS:
                    (A) LENGTH: 36
                    (B) TYPE: Nucleic Acid
                    (C) STRANDEDNESS: Single
                    (D) TOPOLOGY: Linear


TAAAAAGTTA TTTAAGTTAT TCATACTTTC TTCTTC                            36


(2)    INFORMATION FOR SEQ ID NO:60:

        (i)    SEQUENCE CHARACTERISTICS:
                    (A) LENGTH: 34
                    (B) TYPE: Nucleic Acid
                    (C) STRANDEDNESS: Single
                    (D) TOPOLOGY: Linear


GAGTTACCCC TTTCAAAATC ATTTCAGTTA GCAG                              34

(2)    INFORMATION FOR SEQ ID NO:61:

        (i)   SEQUENCE CHARACTERISTICS:
                (A) LENGTH: 56
                (B) TYPE: Nucleic Acid
                (C) STRANDEDNESS: Single
                (D) TOPOLOGY: Linear


GCGACCGGTC GCCGGACGCC TCATACTTTC TTCTTCTTTT CTTTTTTGCT ATAGAA        56


(2)    INFORMATION FOR SEQ ID NO:62:

        (i)   SEQUENCE CHARACTERISTICS:
                (A) LENGTH: 50
                (B) TYPE: Nucleic Acid
                (C) STRANDEDNESS: Single
                (D) TOPOLOGY: Linear


GCGACCGGTC GCCGGACGCC TCTTCTTCTT TTCTTTTTTG CTATAGAAAG        50


(2)    INFORMATION FOR SEQ ID NO:63:

        (i)   SEQUENCE CHARACTERISTICS:
                (A) LENGTH: 49
                (B) TYPE: Nucleic Acid
                (C) STRANDEDNESS: Single
                (D) TOPOLOGY: Linear


GCGACCGGTC GCCGGACGCC GATCACTCCA CTGTTCATAG GGATCCACG        49

(2)    INFORMATION FOR SEQ ID NO:64:

      (i)    SEQUENCE CHARACTERISTICS:

             (A) LENGTH: 49

             (B) TYPE: Nucleic Acid

             (C) STRANDEDNESS: Single

             (D) TOPOLOGY: Linear

GCGACCGGTC GCCGGACGCC GATCACTCCA CTGTTCATAG GGATCCACC    49

(2)    INFORMATION FOR SEQ ID NO:65:

      (i)    SEQUENCE CHARACTERISTICS:

             (A) LENGTH: 30

             (B) TYPE: Nucleic Acid

             (C) STRANDEDNESS: Single

             (D) TOPOLOGY: Linear

CACATATGGT ATGACCCTCT ATATAAACTC    30

(2)    INFORMATION FOR SEQ ID NO:66:

      (i)    SEQUENCE CHARACTERISTICS:

             (A) LENGTH: 30

             (B) TYPE: Nucleic Acid

             (C) STRANDEDNESS: Single

             (D) TOPOLOGY: Linear

TGCCATGGGG CCTGTGCAAG GAAGTATTCC    30

(2)    INFORMATION FOR SEQ ID NO:67:

    (i)    SEQUENCE CHARACTERISTICS:
              (A) LENGTH: 30
              (B) TYPE: Nucleic Acid
              (C) STRANDEDNESS: Single
              (D) TOPOLOGY: Linear


TGCCATGGGG CCTGTGCAAG GAAGTATTCA              30


(2)    INFORMATION FOR SEQ ID NO:68:

    (i)    SEQUENCE CHARACTERISTICS:
              (A) LENGTH: 30
              (B) TYPE: Nucleic Acid
              (C) STRANDEDNESS: Single
              (D) TOPOLOGY: Linear


TGCCATGGGG CCTGTGCAAG GAAGTATTTA              30


(2)    INFORMATION FOR SEQ 1D NO:69:

    (i)    SEQUENCE CHARACTERISTICS:
              (A) LENGTH: 30
              (B) TYPE: Nucleic Acid
              (C) STRANDEDNESS: Single
              (D) TOPOLOGY: Linear


TGCCATGGGG CCTGTGCAAG GAAGTATTGA              30

(2)    INFORMATION FOR SEQ ID NO:70:

        (i)    SEQUENCE CHARACTERISTICS:
                    (A) LENGTH: 30
                    (B) TYPE: Nucleic Acid
                    (C) STRANDEDNESS: Single
                    (D) TOPOLOGY: Linear


TCACATATGG TATGACCCTC TATATAAACT                                    30


(2)    INFORMATION FOR SEQ ID NO:71:

        (i)    SEQUENCE CHARACTERISTICS:
                    (A) LENGTH: 28
                    (B) TYPE: Nucleic Acid
                    (C) STRANDEDNESS: Single
                    (D) TOPOLOGY: Linear


GTCTTTCTCT GCAAACTTGG AGATGTTC                                      28


(2)    INFORMATION FOR SEQ ID NO:72:

        (i)    SEQUENCE CHARACTERISTICS:
                    (A) LENGTH: 28
                    (B) TYPE: Nucleic Acid
                    (C) STRANDEDNESS: Single
                    (D) TOPOLOGY: Linear


GTCTTTCTCT GCAAACTTGG AGATGTAC                                      28

(2)    INFORMATION FOR SEQ ID NO:73:

    (i)   SEQUENCE CHARACTERISTICS:
             (A) LENGTH: 28
             (B) TYPE: Nucleic Acid
             (C) STRANDEDNESS: Single
             (D) TOPOLOGY: Linear

GTCTTTCTCT GCAAACTTGG AGATGTGC                                          28

(2)    INFORMATION FOR SEQ ID NO:74:

    (i)   SEQUENCE CHARACTERISTICS:
             (A) LENGTH: 28
             (B) TYPE: Nucleic Acid
             (C) STRANDEDNESS: Single
             (D) TOPOLOGY: Linear

GTCTTTCTCT GCAAACTTGG AGATGTTT                                          28

(2)    INFORMATION FOR SEQ ID NO:75:

    (i)   SEQUENCE CHARACTERISTICS:
             (A) LENGTH: 28
             (B) TYPE: Nucleic Acid
             (C) STRANDEDNESS: Single
             (D) TOPOLOGY: Linear

GTCTTTCTCT GCAAACTTGG AGATGTGT                                          28

(2)    INFORMATION FOR SEQ ID NO:76:

        (i)   SEQUENCE CHARACTERISTICS:
                    (A) LENGTH: 28
                    (B) TYPE: Nucleic Acid
                    (C) STRANDEDNESS: Single
                    (D) TOPOLOGY: Linear


GTCTTTCTCT GCAAACTTGG AGATGTAT                            28


(2)    INFORMATION FOR SEQ ID NO:77:

        (i)   SEQUENCE CHARACTERISTICS:
                    (A) LENGTH: 30
                    (B) TYPE: Nucleic Acid
                    (C) STRANDEDNESS: Single
                    (D) TOPOLOGY: Linear


AAAATTTCAG CAATGTTGTT TTTGACCAAC                          30


(2)    INFORMATION FOR SEQ ID NO:78:

        (i)   SEQUENCE CHARACTERISTICS:
                    (A) LENGTH: 29
                    (B) TYPE: Nucleic Acid
                    (C) STRANDEDNESS: Single
                    (D) TOPOLOGY: Linear


CCTGTGGTAT CACTCCAAAG GCTTTCCCC                           29

(2)    INFORMATION FOR SEQ ID NO:79:

     (i)   SEQUENCE CHARACTERISTICS:
          (A) LENGTH: 29
          (B) TYPE: Nucleic Acid
          (C) STRANDEDNESS: Single
          (D) TOPOLOGY: Linear

CCTGTGGTAT CACTCCAAAG GCTTTCCCT                                            29


(2)    INFORMATION FOR SEQ ID NO:80:

     (i)   SEQUENCE CHARACTERISTICS:
          (A) LENGTH: 29
          (B) TYPE: Nucleic Acid
          (C) STRANDEDNESS: Single
          (D) TOPOLOGY: Linear

CCTGTGGTAT CACTCCAAAG GCTTTCCGT                                            29


(2)    INFORMATION FOR SEQ ID NO:81:

     (i)   SEQUENCE CHARACTERISTICS:
          (A) LENGTH: 29
          (B) TYPE: Nucleic Acid
          (C) STRANDEDNESS: Single
          (D) TOPOLOGY: Linear

CCTGTGGTAT CACTCCAAAG GCTTTCCGC                                            29

(2)    INFORMATION FOR SEQ ID NO:82:

      (i)    SEQUENCE CHARACTERISTICS:

              (A) LENGTH: 29

              (B) TYPE: Nucleic Acid

              (C) STRANDEDNESS: Single

              (D) TOPOLOGY: Linear

CCTGTGGTAT CACTCCAAAG GCTTTCCAC                29

(2)    INFORMATION FOR SEQ ID NO:83:

      (i)    SEQUENCE CHARACTERISTICS:

              (A) LENGTH: 29

              (B) TYPE: Nucleic Acid

              (C) STRANDEDNESS: Single

              (D) TOPOLOGY: Linear

CCTGTGGTAT CACTCCAAAG GCTTTCCAT                29

(2)    INFORMATION FOR SEQ ID NO:84:

      (i)    SEQUENCE CHARACTERISTICS:

              (A) LENGTH: 30

              (B) TYPE: Nucleic Acid

              (C) STRANDEDNESS: Single

              (D) TOPOLOGY: Linear

CCCATCACTT TTACCTTATA GGTGGGCCTC                30

(2)    INFORMATION FOR SEQ ID NO:85:

     (i)    SEQUENCE CHARACTERISTICS:

         (A) LENGTH: 30

         (B) TYPE: Nucleic Acid

         (C) STRANDEDNESS: Single

         (D) TOPOLOGY: Linear

GTACCTATAT GTCACAGAAG TGATCCCATC          30

(2)    INFORMATION FOR SEQ ID NO:86:

     (i)    SEQUENCE CHARACTERISTICS:

         (A) LENGTH: 35

         (B) TYPE: Nucleic Acid

         (C) STRANDEDNESS: Single

         (D) TOPOLOGY: Linear

CGGCGATGTT TTTTCTGGAG ATTTATGTTC TATTG     35

(2)    INFORMATION FOR SEQ ID NO:87:

     (i)    SEQUENCE CHARACTERISTICS:

         (A) LENGTH: 35

         (B) TYPE: Nucleic Acid

         (C) STRANDEDNESS: Single

         (D) TOPOLOGY: Linear

CGGCGATGTT TTTTCTGGAG ATTTATGTTC TATTA     35

(2)    INFORMATION FOR SEQ ID NO:88:

(i)   SEQUENCE CHARACTERISTICS:
                (A) LENGTH: 35
                (B) TYPE: Nucleic Acid
                (C) STRANDEDNESS: Single
                (D) TOPOLOGY: Linear

CGGCGATGTT TTTTCTGGAG ATTTATGTTC TATAG                              35

(2)    INFORMATION FOR SEQ ID NO:89:

(i)   SEQUENCE CHARACTERISTICS:
                (A) LENGTH: 35
                (B) TYPE: Nucleic Acid
                (C) STRANDEDNESS: Single
                (D) TOPOLOGY: Linear

CGGCGATGTT TTTTCTGGAG ATTTATGTTC TATAA                              35

(2)    INFORMATION FOR SEQ ID NO:90:

(i)   SEQUENCE CHARACTERISTICS:
                (A) LENGTH: 31
                (B) TYPE: Nucleic Acid
                (C) STRANDEDNESS: Single
                (D) TOPOLOGY: Linear

ATTCACCAGA TTTCGTAGTC TTTTCATAATC                                   31

(2)    INFORMATION FOR SEQ ID NO:91:

        (i)   SEQUENCE CHARACTERISTICS:
                    (A) LENGTH: 30
                    (B) TYPE: Nucleic Acid
                    (C) STRANDEDNESS: Single
                    (D) TOPOLOGY: Linear


CCCACCTTCC CCTCTCTCCA GGCAAATGGG                                    30


(2)    INFORMATION FOR SEQ ID NO:92:

        (i)   SEQUENCE CHARACTERISTICS:
                    (A) LENGTH: 30
                    (B) TYPE: Nucleic Acid
                    (C) STRANDEDNESS: Single
                    (D) TOPOLOGY: Linear


GGGCCTCAGT CCCAACATGG CTAAGAGGTG                                    30


(2)    INFORMATION FOR SEQ ID NO:93:

        (i)   SEQUENCE CHARACTERISTICS:
                    (A) LENGTH: 30
                    (B) TYPE: Nucleic Acid
                    (C) STRANDEDNESS: Single
                    (D) TOPOLOGY: Linear


TGTCCACGTG AGCCTTGCTC GAGGCCTGGG                                    30

(2) INFORMATION FOR SEQ ID NO:94:

    (i) SEQUENCE CHARACTERISTICS:
        (A) LENGTH: 29
        (B) TYPE: Nucleic Acid
        (C) STRANDEDNESS: Single
        (D) TOPOLOGY: Linear

GAGACTTGGT ATTTTGTTCA ATCATTAAG         29

(2) INFORMATION FOR SEQ ID NO:95:

    (i) SEQUENCE CHARACTERISTICS:
        (A) LENGTH: 20
        (B) TYPE: Nucleic Acid
        (C) STRANDEDNESS: Single
        (D) TOPOLOGY: Linear

TCATGCGTCC ATGGTCCGGA         20

(2) INFORMATION FOR SEQ ID NO:96:

    (i) SEQUENCE CHARACTERISTICS:
        (A) LENGTH: 30
        (B) TYPE: Nucleic Acid
        (C) STRANDEDNESS: Single
        (D) TOPOLOGY: Linear

GACTGACTGA CTGACTGACT CTGACTGACT         30

(2)    INFORMATION FOR SEQ ID NO:97:

    (i)   SEQUENCE CHARACTERISTICS:
                 (A) LENGTH: 20
                 (B) TYPE: Nucleic Acid
                 (C) STRANDEDNESS: Single
                 (D) TOPOLOGY: Linear

GCGACCGGTC GCCGGACGCC                                           20

## Claims

1. A method for detecting the presence or absence of more than one variant nucleotide in a target base sequence comprised in a nucleic acid sample, which method comprises treating the target base sequence, together or sequentially with appropriate nucleoside triphosphates, an agent for polymerisation of the nucleoside triphosphates and a diagnostic primer for each diagnostic portion of the target base sequence under hybridising conditions, the nucleotide sequence of each diagnostic primer being such that it is substantially complementary to the relevant diagnostic portion, a terminal nucleotide of each diagnostic primer being either complementary to a suspected variant nucleotide or to the corresponding normal nucleotide, whereby an extension product of a diagnostic primer is synthesised when the terminal nucleotide of the diagnostic primer is complementary to the corresponding nucleotide in the target base sequence, no extension product being synthesised when the terminal nucleotide of a diagnostic primer is not complementary to the corresponding nucleotide in the target base sequence; and detecting the presence or absence of the suspected variant nucleotides from the presence or absence of extension products and wherein the extension products of diagnostic primer(s) for more than one diagnostic portion of the target base sequence comprise a complementary overlap.

2. A method as claimed in claim 1 wherein at least two of the diagnostic primers comprise a complementary overlap.

3. A method as claimed in claim 1 or claim 2 wherein a corresponding amplification primer is used, any extension product of a diagnostic primer being capable of serving as a template for synthesis of an extension product of the amplification primer after separation from its complement.

4. A method for detecting the presence or absence of variant nucleotides in first and second target base sequences comprised in a nucleic acid sample, which method comprises treating the target base sequences, together or sequentially with appropriate nucleoside triphosphates, an agent for polymerisation of the nucleoside triphosphates and a first diagnostic primer for a diagnostic portion of the first target base sequence and a second diagnostic primer for a diagnostic portion of the second target base sequence under hybridising conditions, the nucleotide sequence of each diagnostic primer being such that it is substantially complementary to the relevant diagnostic portion, a terminal nucleotide of each diagnostic primer being either complementary to a suspected variant nucleotide or to the corresponding normal nucleotide, whereby an extension product of a diagnostic primer is synthesised when the terminal nucleotide of the diagnostic primer is complementary to the corresponding nucleotide in the target base sequence, no extension product being synthesised when the terminal nucleotide of a diagnostic primer is not complementary to the corresponding nucleotide in the target base sequence; and wherein corresponding amplification primers are used, any extension product of a diagnostic primer being capable of serving as a template for synthesis of an extension product of the corresponding amplification primer after separation from its complement and detecting the presence or absence of the suspected variant nucleotides from the presence or absence of amplification products of each diagnostic primer and corresponding amplification primer and wherein the amplification products comprise a complementary overlap.

5. A method as claimed in claim 4 wherein any of the diagnostic and/or amplification primers comprise a complementary overlap.

6. A method as claimed in claim 4 wherein the first and second diagnostic primers comprise a complementary overlap.

7. A method for detecting the presence or absence of at least one variant nucleotide in a target base sequence comprised in a nucleic acid sample, which method comprises treating the target base sequence, together or sequentially with appropriate nucleoside triphosphates, an agent for polymerisation of the nucleoside triphosphates and a diagnostic primer for a diagnostic portion of the target base sequence under hybridising conditions, the nucleotide sequence of the diagnostic primer being such that it is substantially complementary to the diagnostic portion, a terminal nucleotide of the diagnostic primer being either complementary (i) to at least two suspected variant nucleotides or (ii) to at least two corresponding normal nucleotides; whereby an extension product of the diagnostic primer is synthesised when the terminal nucleotide of the diagnostic primer is complementary to at least one corresponding variant or normal nucleotide in the target base sequence; no extension product being synthesised when the terminal nucleotide of the diagnostic primer is non-complementary to all suspected variant nucleotides or non-complementary to all corresponding normal nucleotides in the target base sequence; and detecting the presence or absence of the suspected variant nucleotides from the presence or absence of extension products.

8. A method as claimed in claim 7 wherein a corresponding amplification primer is used, any extension product of the diagnostic primer being capable of serving as a template for synthesis of an extension product of the amplification primer after separation from its complement.

9. A method as claimed in claim 7 wherein the nucleotide sequence of the diagnostic primer allows internal hybridisation adjacent the terminal diagnostic nucleotide.

10. A method as claimed in any one of the previous claims wherein at least one of the diagnostic and/or amplification primers comprises a polynucleotide tail sequence which do not hybridise to the target base sequence.

11. A method as claimed in any one of claims 1-10 wherein at least three suspected variant nucleotides are analysed.

12. A set of at least two nucleotide primers of 5-50 bp and as defined in claim 1, a terminal nucleotide of each primer being complementary to either a suspected variant nucleotide associated with a known genetic disorder or to the corresponding normal nucleotide.

13. A diagnostic primer as defined in claim 7 or claim 9.

14. A kit for performing a method as claimed in claim 1 which comprises:
    (1) diagnostic primers as defined in claim 1;
    (2) each of four different nucleoside triphosphates; and
    (3) an agent for polymerisation of the nucleoside triphosphates in (2).

15. A kit for performing a method as claimed in claim 4 which comprises:
    (1) diagnostic primers as defined in claim 4;
    (2) each of four different nucleoside triphosphates;
    (3) an agent for polymerisation of the nucleoside triphosphates in (2); and
    (4) corresponding amplification primers as defined in claim 4.

16. A kit for performing a method as claimed in claim 7 which comprises:
    (1) a diagnostic primer as defined in claim 7;
    (2) each of four different nucleoside triphosphates;
    (3) an agent for polymerisation of the nucleoside triphosphates in (2).

17. A kit as claimed in claim 14 or claim 16 and further comprising a corresponding amplification primer as defined in claim 3 or claim 8.

18. A kit as claimed in any one of claims 14-17 and further comprising one or more of the following items: internal control primers, buffer, packaging and instructions for use of the kit.

19. A method as claimed in any one of claims 1-11, primers as claimed in claims 12-13 or a kit as claimed in any one of claims 14-18 for use in the detection or diagnosis of cystic fibrosis.

Fig. 1.

# Fig.1cont.

```
        ...XIII                          XIVa  XIVb    XV...
    ┌─────────────────────────────────────┬────────┬──┬──────────────┐
    └─────────────────────────────────────┴────────┴──┴──────────────┘
    2001                                                          3000

        XVI   XVIIa      XVIIb         XVIII    XIX        XX...
    ┌───┬──────┬───────────────────┬─────────┬──────────┬──────────┐
    └───┴──────┴───────────────────┴─────────┴──────────┴──────────┘
    3001        ╱ N1303K    (C-G 4041)                          4000
              XXI   XXII   XXIII    XXIV
    ┌────┬────────┬─────────┬──────────────┐
    └────┴────────┴─────────┴──────────────┘
    4001                                    4573 TAG
    1867C
          1852N>
          1851M>
          1870N>   <1868N
          1871M>   <1869M
          1872N>                    <1850C
          1873M> 1885M > 1894M>        <1874C
                                     21/22 intron
```

*Fig.2.*

M. 1 . 2 . 3 . 4 . 5 . c . 6 . 7 . 8

$=_{C}^{C}$ F508

*Fig.4.*

1 . 2 . 3 . 4 . 5 . 6 . c . m

R560T—
G542X—

*Fig.5.*

1 . 2 . 3

—R560T

—F508

*Fig.6.*

. 1 . 2 . 3 . 4 . 5 . 6 . 7 . * . 8 . * . cm

b $\frac{a}{c}$
d

*Fig.3(a)*

```
                        3'TTACCACAAAGGAT...... 508M
                   <-  3'ACACCACAAAGGAT.......508N

           5' GAAAATATCATCTTTGGTGTT
              CTTTTATAGTAGAAACCAGAA 5'

    508/507N......GAAAATATCATCTT 3' ->
    508/507M......GAAAATATCATTGG 3,
```

*Fig.3(b)*

```
                   <-  3'TTACCACAAAGGAT...... 508M
                       3'ACACCACAAAGGAT...... 508N

           5' GAAAATATCATTGGTGTTTCC
              CTTTTATAGTAACCACAAAGG 5'

    508/507N.....GAAAATATCATCTT 3'
    508/507M.....GAAAATATCATTGG 5' ->
```

*Fig.3(c)*

```
                       3'TTACCACAAAGGAT...... 508M
                   <-  3'ACACCACAAAGGAT...... 508N

           5' GAAAATATCTTTGGTGTTTCC
              CTTTTATAGAAACCACAAAGG 5'

    508/507N.....GAAAATATCATCTT 3'
    508/507M.....GAAAATATCATTGG 3' ->
```

76

## Fig. 7.

## Fig.8.

| sample | 621 + 1G >T | G551D | G542X | F508 | g |
|--------|-------------|-------|-------|------|---|
| 1 | n/n | n/n | n/n | n/n | n |
| 2 | n/n | n/n | n/n | n/n | n |
| 3 | n/n | n/n | n/n | n/n | n |
| 4 | n/n | n/n | n/n | n/m | F508 h |
| 5 | n/n | n/n | n/m | n/m | G542X/F508 |
| 6 | n/n | n/m | n/n | n/m | G551D/F508 |
| 7 | n/m | n/n | n/n | n/m | 621+1/F508 |
| 8 | n/n | m/m | n/n | n/n | G551D H |

## Fig. 9(i)

DP1  DP2

## Fig.9(ii)

DP1

DP2

## Fig.9(iii)

DP2
DP1

## Fig.9(iv)

DP3
DP2
DP1

*Fig.10(i)*

AP2      DP1

DP2

AP1

*Fig.10(ii)*

AP2      DP1

DP2

AP1

*Fig.10(iii)*

DP2

DP1

AP 1-2

*Fig.10(iv)*

DP3

DP2

DP1

AP 1-3

*Fig. 11A*

*Fig. 11B*

*Fig. 11C*

*Fig. 11D*

*Fig.12.*

621+1G>T
G551D
G542X
F508

$a_1^1 b \quad a_1^2 b \quad a_1^3 b \quad a_1^4 b \quad a_1^5 b \quad a_1^6 b$

*Fig.13.*

621+1G>T
R553X
G551D
G542X
W1282X
F508

$a_1^1 b \quad a_1^2 b \quad a_1^3 b \quad a_1^4 b \quad a_1^5 b \quad a_1^6 b \quad a_1^7 b \quad$ blank

*Fig.14.*

R560T
R117H
1717G>A
I507/F508
N1303K

$a_1^1 b \quad a_1^2 b \quad a_1^3 b \quad a_1^4 b \quad a_1^5 b \quad a_1^6 b \quad a_1^7 b$

## Fig. 15(a) F508

N   F508   I507

Np

Mp

1 2 3 4 5

C
AP

160/163bp

## Fig. 15(b) I507 / F508

N   F508   I507

Np

Mp

1 2 3 4 5

C
AP

146/149bp

EP 0 497 527 A1

<table>
<tr><td colspan="5" align="center">**EUROPEAN SEARCH REPORT**</td></tr>
</table>

European Patent
Office

**EUROPEAN SEARCH REPORT**

Application Number

EP   92 30 0660

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-9 011 372 (COLLABORATIVE RESEARCH INC.)<br><br>* the whole document *<br>*ESPECIALLY PAGE 22 LINE 23-PAGE 28*<br>--- | 1,2,10,<br>11,12,<br>14,17-19 | C12Q1/68<br>C12P19/34<br>//C07H21/04 |
| X | EP-A-0 317 239 (NATIVE PLANTS INC.)<br>* the whole document *<br>*especially page 9, lines 9-36; claims 20-22* | 4,5,15 | |
| A | | 1,3,11,<br>12,14,<br>17-19 | |
| X | WO-A-8 910 414 (WALLACE ET AL.)<br>* the whole document *<br>*especially claims 23 and 24* | 4,5,15 | |
| A | | 1,3,11,<br>12,14,<br>17-19 | |
| D,A | EP-A-0 332 435 (IMPERIAL CHEMICAL INDUSTRIES INC.)<br>* the whole document *<br>--- | 1-19 | TECHNICAL FIELDS SEARCHED (Int. Cl.5) |
| A | WO-A-9 009 455 (GENECO PTY LTD.)<br>* the whole document *<br>*especially claims 6 and 43*<br>--- | 1-19 | C12Q |
| A | EP-A-0 364 255 (BAYLOR COLLEGE OF MEDICINE)<br>* the whole document *<br>*especially pages 17 and 18*<br>----- | 1,4 | |

The present search report has been drawn up for all claims

| Place of search | Date of completion of the search | Examiner |
|---|---|---|
| THE HAGUE | 08 MAY 1992 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)